# EUROPEAN PATENT SPECIFICATION

(11) **EP 1 481 671 B1**
(45) Date of publication and mention of the grant of the patent: **31.08.2011**
(21) Application number: 03705174.5
(22) Date of filing: 14.02.2003
(51) Int. Cl.: A61K 31/122, A61P 3/04, A61P 3/06, A61P 3/10, A61P 43/00, A23L 1/30, A61K 36/185

(54) **COMPOSITIONS AND FOODS FOR IMPROVING LIPID METABOLISM**
ZUSAMMENSETZUNGEN UND NAHRUNGSMITTEL ZUR VERBESSERUNG DES FETTSTOFFWECHSELS
COMPOSITIONS ET ALIMENTS AMELIORANT LE METABOLISME DES LIPIDES

(30) Priority: 14.02.2002 JP 2002036798; 15.05.2002 JP 2002139700
(43) Date of publication of application: 01.12.2004
(73) Proprietor: KIRIN BEER KABUSHIKI KAISHA, Tokyo 104-8288 (JP)
(72) Inventor: YAJIMA, Hiroaki, Yokohama-shi, Kanagawa (JP); MIURA, Yutaka, Yokohama-shi, Kanagawa (JP); FUJIWARA, Daisuke, Yokohama-shi, Kanagawa (JP); ODAI, Hideharu, Yokohama-shi, Kanagawa-Shi (JP); KONDO, Keiji, Tokyo 104-8288 (JP); NOZAWA, Hajime c/o Kirin Beer Kabushiki Kaisha, Gumma-ken (JP)
(74) Representative: HOFFMANN EITLE
(86) International application number: PCT/JP2003/001571
(87) International publication number: WO 2003/068205

(56) References cited:
- EP-A2- 0 677 289
- WO-A-00/27832
- WO-A1-00/62772
- WO-A1-01/14351
- WO-A2-03/035007
- FR-A- 1 164 537
- JP-A- 11 335 231
- JP-A- 50 070 512
- JP-A- 59 059 623
- JP-A- 2001 131 080
- JP-A- 2001 226 274
- JP-A- 2001 261 612
- JP-A- 2001 321 166
- JP-A- 2001 354 558
- US-A- 2 898 374
- DAISUKE FUJIWARA ET AL.: 'Beer nigami seibun no PPARgamma kasseika sayo' 2J-01P, THE JAPANESE SOCIETY OF NUTRITION AND FOOD SCIENCE SOGO KOEN YOSHISHU vol. 56, 2002, page 161, XP002975352
- HIROAKI YAJIMA ET AL.: 'Beer nigami seibun no insulin teikosei kaizen sayo' SJ-02P, THE JAPANESE SOCIETY OF NUTRITION AND FOOD SCIENCE SOHO KOEN YOSHISHU vol. 56, 2002, page 61, XP002975353
- HIROSHI MIURA ET AL.: 'Beer nigami seibun no shishitsu taisha kaiten koka (dai 1 ho)' 3M-02A, THE JAPANESE SOCIETY OF NUTRITION AND FOOD SCIENCE SOGO KOEN YOSHISHU vol. 56, 2002, page 353, XP002975354
- TSUJIHARA K. ET AL.: 'Na+-glucose cotransporter (SGLT) inhibitors as antidiabetic agents. 4. Synthesis and pharmacological properties of 4'-dehydroxyphlorizin derivatives substituted on the B. ring' J. MED. CHEM. vol. 42, no. 26, 1999, pages 5311 - 5324, XP002955098

### SEQUENCE LISTING

<110> Kirin Beer Kabushiki Kaisha
<120> Compositions and foods for improving lipid metabolism
<130> 140875PX
<140>
   <141>
<150> 36798/2002 <151> 2002-02-14
<150> 139700/2002 <151> 2002-05-15
<160> 36
<170> PatentIn Ver. 2.1
<210> 1
   <211> 24
   <212> DNA
   <213> Artificial Sequence
<220>
   <223> Description of Artificial Sequence: PCR primer
<400> 1
   atctatgacc aggttcagtc gggg 24
<210> 2
   <211> 22
   <212> DNA
   <213> Artificial Sequence
<220>
   <223> Description of Artificial Sequence: PCR primer
<400> 2
   ccacgccact tccttgctct tc 22
<210> 3
   <211> 22
   <212> DNA
   <213> Artificial Sequence
<220>
   <223> Description of Artificial Sequence: PCR primer
<400> 3
   ggaactacag gcaaccccaa ag 22
<210> 4
   <211> 22
   <212> DNA
   <213> Artificial Sequence
<220>
   <223> Description of Artificial Sequence: PCR primer
<400> 4
   cttgaggtcg tccataagca gc 22
<210> 5
   <211> 21
   <212> DNA
   <213> Artificial Sequence
<220>
   <223> Description of Artificial Sequence: PCR primer
<400> 5
   tgctagtgat ggacgagctg g 21
<210> 6
   <211> 24
   <212> DNA
   <213> Artificial Sequence
<220>
   <223> Description of Artificial Sequence: PCR primer
<400> 6
   tcctggtaca ttgagttagg gtcc 24
<210> 7
   <211> 24
   <212> DNA
   <213> Artificial Sequence
<220>
   <223> Description of Artificial Sequence: PCR primer
<400> 7
   ccttcagggg tctaaagctg gaag 24
<210> 8
   <211> 22
   <212> DNA
   <213> Artificial Sequence
<220>
   <223> Description of Artificial Sequence: PCR primer
<400> 8
   cagccaattc ttgggcagag tg 22
<210> 9
   <211> 20
   <212> DNA
   <213> Artificial Sequence
<220>
   <223> Description of Artificial Sequence: PCR primer
<400> 9
   ttggcctcca ttgagatccg 20
<210> 10
   <211> 22
   <212> DNA
   <213> Artificial Sequence
<220>
   <223> Description of Artificial Sequence: PCR primer
<400> 10
   gatcttgttg ttgccggtga ac 22
<210> 11
   <211> 23
   <212> DNA
   <213> Artificial Sequence
<220>
   <223> Description of Artificial Sequence: PCR primer
<400> 11
   catcaaggag tgcaagacca acg 23
<210> 12
   <211> 24
   <212> DNA
   <213> Artificial Sequence
<220>
   <223> Description of Artificial Sequence: PCR primer
<400> 12
   cacttgtagc tgccttccag gttc 24
<210> 13
   <211> 23
   <212> DNA
   <213> Artificial Sequence
<220>
   <223> Description of Artificial Sequence: PCR primer
<400> 13
   tgtatgtgga tgcggtcaaa gac 23
<210> 14
   <211> 24
   <212> DNA
   <213> Artificial Sequence
<220>
   <223> Description of Artificial Sequence: PCR primer
<400> 14
   tcatctcctg tctcacccaa tctg 24
<210> 15
   <211> 22
   <212> DNA
   <213> Artificial Sequence
<220>
   <223> Description of Artificial Sequence: PCR primer
<400> 15
   agggctacat ggaacaagcc tc 22
<210> 16
   <211> 24
   <212> DNA
   <213> Artificial Sequence
<220>
   <223> Description of Artificial Sequence: PCR primer
<400> 16
   cgactcaata gctggagttg gttg 24
<210> 17
   <211> 22
   <212> DNA
   <213> Artificial Sequence
<220>
   <223> Description of Artificial Sequence: PCR primer
<400> 17
   gtttggctcc agagtttgac cg 22
<210> 18
   <211> 24
   <212> DNA
   <213> Artificial Sequence
<220>
   <223> Description of Artificial Sequence: PCR primer
<400> 18
   catacattcc cgttaccgtc catc 24
<210> 19
   <211> 22
   <212> DNA
   <213> Artificial Sequence
<220>
   <223> Description of Artificial Sequence: PCR primer
<400> 19
   acgggttgat tccatacctg gg 22
<210> 20
   <211> 21
   <212> DNA
   <213> Artificial Sequence
<220>
   <223> Description of Artificial Sequence: PCR primer
<400> 20
   tgtgtccaaa tgccttcgca g 21
<210> 21
   <211> 21
   <212> DNA
   <213> Artificial Sequence
<220>
   <223> Description of Artificial Sequence: PCR primer
<400> 21
   ccaagcagat gcagcagatc c 21
<210> 22
   <211> 21
   <212> DNA
   <213> Artificial Sequence
<220>
   <223> Description of Artificial Sequence: PCR primer
<400> 22
   cagcagctgg caccttattg g 21
<210> 23
   <211> 24
   <212> DNA
   <213> Artificial Sequence
<220>
   <223> Description of Artificial Sequence: PCR primer
<400> 23
   cgtgggacat tcgtgaagaa aaag 24
<210> 24
   <211> 21
   <212> DNA
   <213> Artificial Sequence
<220>
   <223> Description of Artificial Sequence: PCR primer
<400> 24
   tgtgcttgtg tgtggattcg c 21
<210> 25
   <211> 21
   <212> DNA
   <213> Artificial Sequence
<220>
   <223> Description of Artificial Sequence: PCR primer
<400> 25
   ccaagcagat gcagcagatc c 21
<210> 26
   <211> 21
   <212> DNA
   <213> Artificial Sequence
<220>
   <223> Description of Artificial Sequence: PCR primer
<400> 26
   cagcagctgg caccttattg g 21
<210> 27
   <211> 26
   <212> DNA
   <213> Artificial Sequence
<220>
   <223> Description of Artificial Sequence: PCR primer
<400> 27
   gctagcatgg tggacacgga aagccc 26
<210> 28
   <211> 28
   <212> DNA
   <213> Artificial Sequence
<220>
   <223> Description of Artificial Sequence: PCR primer
<400> 28
   gtcgacagta catgtccctg tagatctc 28
<210> 29
   <211> 55
   <212> DNA
   <213> Artificial Sequence
<220>
   <223> Description of Artificial Sequence: PCR primer
<400> 29
   caggggacca ggacaaaggt cacgttcggg aaggggacca ggacaaaggt cacgt 55
<210> 30
   <211> 55
   <212> DNA
   <213> Artificial Sequence
<220>
   <223> Description of Artificial Sequence: PCR primer
<400> 30
   gatcttcccg aacgtgacct ttgtcctggt ccccttcccg aacgtgacct ttgtc 55
<210> 31
   <211> 30
   <212> DNA
   <213> Artificial Sequence
<220>
   <223> Description of Artificial Sequence: PCR primer
<400> 31
   ggatcctttc tcataatgcc atcaggtttg 30
<210> 32
   <211> 27
   <212> DNA
   <213> Artificial Sequence
<220>
   <223> Description of Artificial Sequence: PCR primer
<400> 32
   ggtaccttcc gtgacaatct gtctgag 27
<210> 33
   <211> 29
   <212> DNA
   <213> Artificial Sequence
<220>
   <223> Description of Artificial Sequence: PCR primer
<400> 33
   gctagcatgg actccaaaga atcattaac 29
<210> 34
   <211> 20
   <212> DNA
   <213> Artificial Sequence
<220>
   <223> Description of Artificial Sequence: PCR primer
<400> 34
   tggctgctgc gcattgctta 20
<210> 35
   <211> 30
   <212> DNA
   <213> Artificial Sequence
<220>
   <223> Description of Artificial Sequence: PCR primer
<400> 35
   ggatcctttc acacaacgcg attcgttttg 30
<210> 36
   <211> 27
   <212> DNA
   <213> Artificial Sequence
<220>
   <223> Description of Artificial Sequence: PCR primer
<400> 36
   ggtaccgtac atgtccctgt agatctc 27

## Description

### BACKGROUND OF THE INVENTION

### Field of the Invention

The present invention relates to pharmaceutical compositions for use in ameliorating insulin resistance, improving lipid metabolism, suppressing body weight gain, or slimming. The present invention also relates to foods containing these pharmaceutical compositions.

### Background Art

In recent years, owing to the westernization of eating habits in Japan, fat intake per person has been rising and so-called lifestyle diseases, such as diabetes, hyperlipidemia, hypertension, and obesity, have drastically increased. These diseases tend to develop in unison, and the presence of insulin resistance is considered to have a great influence on this development.

As reported by Yamada et al., patients with impaired glucose tolerance often suffer from such complications as hypertriglycemia, hypercholesterolemia, and hypo-HDL-cholesterolemia (Diabetes Care 17:107-114, 1994). Reaven called a group of symptoms including impaired glucose tolerance caused by insulin resistance, hypertension, hyper-VLDL-cholesterolemia, and hypo-HDL-cholesterolemia "Syndrome x," and suggested that amelioration of these symptoms is important in preventing cerebrovascular disorders and coronary artery diseases (Diabetes 37:1595-1607, 1988) . Thus, the so-called lifestyle diseases, such as diabetes, hyperlipidemia, and hypertension, tend to converge on a patient and are accordingly called multiple risk factors to cause cerebrovascular disorders and coronary artery diseases.

In Japan, the total number of patients with and candidates for diabetes associated with insulin resistance is estimated to exceed 13 million and has consistently been increasing. Excessive excretion of insulin due to insulin resistance is believed to cause increases in LDL cholesterol and triglyceride levels due to lipid metabolism abnormalities and hypertension. Further, an increase in the blood sugar level due to diabetes causes complications such as neural disorders, retinopathies, and renal disorders. Accordingly, development of pharmaceutical compositions for ameliorating insulin resistance and hyperglycemia has become important. Thiazolidine derivatives and the like are known as drugs for ameliorating insulin resistance; however, side effects caused by long-term administration of these drugs, such as an increase in body fat, have been reported and thus development of novel drugs is in demand. Further, since the onset of insulin resistance is closely related to lifestyle, it is also desirable that food or drink having these improving effects can be included in daily meals.

Lipid metabolism abnormalities are caused not only by insulin resistance but also by excessive intake of fat and cholesterol. Increases in LDL cholesterol and triglyceride levels as well as a decrease in HDL cholesterol level in the blood cause arteriosclerosis. The overall mortality rate of arteriosclerosis including ischemic heart disease and cerebrovascular disorders is higher than that of malignant tumors (cancers) and is expected to increase in the future since the amount of fat intake in young people and the amount of animal fat intake in all generations have been markedly increasing. Under these circumstances, there has been a strong need for pharmaceuticals, foods and drinks which are effective for improving lipid metabolism, suppressing lipid accumulation, and further increasing the level of HDL, a so-called beneficial cholesterol, having the capability of removing excess cholesterol from peripheral tissues. Conventionally, polyvalent unsaturated fatty acid such as linoleic acid, a fibrate drug and nicotinic acid are known to be used as an agent for improving lipid metabolism. However, disadvantageously, polyvalent unsaturated fatty acid needs to be taken continuously for a long period of time and causes problems when taken excessively; a fibrate drug causes side effects such as muscle spasms; and nicotinic acid intake also causes undesirable side effects such as systemic flush and gastrointestinal disorders.

Examples of drugs for improving symptoms of insulin resistance and lipid metabolism abnormalities include thiazolidine derivatives (e.g., pioglitazone, troglitazone) and fibrate drugs (e.g., phenofibrate, bezafibrate), which are shown to act as a PPAR agonist. The target of the former compounds is y type (referred to as "PPARγ" hereinafter) mainly distributed in fat tissues, and the target of the latter is α type (referred to as "PPARα" hereinafter) present in the liver, kidney, heart, and alimentary tract.

The hop (Humulus lupulus) is a native European perennial which belongs to the family Cannabaceae, and its fruiting bodies (strobili of female flowers), generally called hops, are widely known to be used for adding a bitter taste and aroma to beer and thus have long been ingested by humans. Such bitter taste and aroma come from hop lupulin (yellow granules formed in the root of the inner scales of strobili). Hops are used also as a folk medicine and known to have various physiological effects, such as inducing sadation, encouraging sleep, inducing sound sleep, stimulating appetite, soothing the stomach, and diuretic effect. Further, their anti-diabetic effect has been also reported (Japanese Patent Laid-open Publication No. 70512/1975, Japanese Patent Laid-open Publication No. 59623/1979). However, it has not been revealed which components of hops are responsible for these physiological actions.

Also, in recent years, it has been reported that polyphenols derived from hop scales obtained from hop corns, from which the lupulin part is removed, have activities such as inhibiting lipase activity and suppressing body weight gain (Japanese Patent Laid-open Publication No. 321166/2001, Japanese Patent Laid-open Publication No. 131080/2001). However, as for humulones and isohumulones that are bitter components of hops, PPAR agonist activity, activity involved in adipocyte differentiation, and activity involved in activation of β-oxidation enzymes, which suggest such agonistic activity, have not been known. Further, it has also not been disclosed that this bitter taste component of hop can ameliorate insulin resistance, improve lipid metabolism such as increasing blood HDL cholesterol or suppressing accumulation of liver lipid, suppress body weight gain, and prevent fat accumulation.

JP 2001-226274 describes an agent for inhibiting lipase or suppressing obesity containing an unisomerized hop extract. JP 11-335231 describes a slimming agent containing an unisomerized hop extract for external use on the body. FR 1 164 537 describes a food comprising a hop extract for controlling body weight. WO 03/035007 describes α-acids such as humulone or β-acids such as lupulone for the treatment of inflammation. JP 2001-354558 is concerned with PPAR activators but does not disclose any of the compositions of the present invention. EP 0 677 289 describes the use of compounds isolated from isomerized hop extracts for the treatment of osteoporosis. US 5370897 sets forth a method for the production of isomerized hop extracts. CN 1269226 relates to the use of beer to treat hypertension, high blood lipids and obesity.

### SUMMARY OF THE INVENTION

The present inventors have found that major bitter taste components of hops, humulones and isomerized compounds thereof, act as an agonist for PPARα and PPARγ. Also, the present inventors have found that these compounds have activities to reduce the free fatty acid concentration, triglyceride concentration, insulin concentration and resistin concentration in the blood, and activities to ameliorate insulin resistance, such as the amelioration of glucose tolerance. Further, the present inventors have found that these compounds have activities for improving lipid metabolism such as increasing the HDL cholesterol level in the blood and suppressing the accumulation of cholesterol and triglyceride in the liver, and suppressing accumulation of visceral fat, and suppressing body weight gain caused by high fat or high cholesterol intake. The present invention is based on these findings.

An object of the present invention is to provide compositions and foods for use in the treatment, prophylaxis, or amelioration of diseases or symptoms which can be treated, prevented or ameliorated by activating PPAR, in particular, insulin resistant diabetes and hyperlipidemia.

Another object of the prevent invention is to provide compositions and foods for use in the amelioration of insulin resistance, the improvement of lipid metabolism, the suppression of body weight gain, the slimming, and the like.

A pharmaceutical composition according to the present invention is for use in the treatment, prophylaxis, or amelioration of diseases or symptoms according to claim 1 comprising
a compound of formula (II) 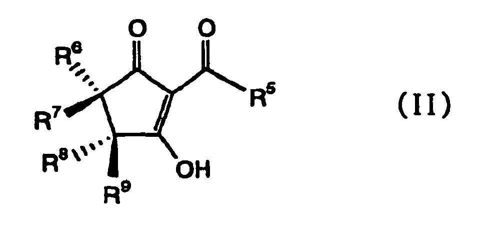 wherein R⁵, R⁶ and R⁷ represent a hydrogen atom, C₁₋₆ alkyl or C₂₋₆ alkenyl, R⁸ and R⁹ represent a hydrogen atom, a hydroxyl group, C₁₋₆ alkyl, C₂₋₆ alkenyl, -C(=O)R¹⁰, or -CH(-OR)R¹⁰, and R¹⁰ represents C₁₋₆ alkyl or C₂₋₆ alkenyl, provided that R⁸ and R⁹ do not simultaneously represent a hydroxyl group;
a compound of formula (III) 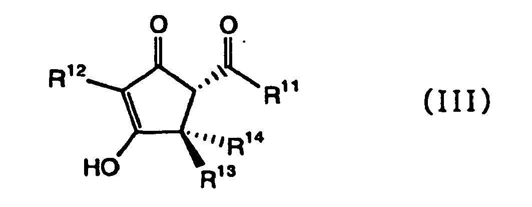 wherein R¹¹ and R¹² represent a hydrogen atom, C₁₋₆ alkyl or C₂₋₆ alkenyl, R¹³ and R¹⁴ represent a hydroxyl group, C₁₋₆ alkyl, C₂₋₆ alkenyl, -C(=O)R¹⁵, or -CH(-OH)R¹⁵, and R¹⁵ represents C₁₋₆ alkyl or C₂₋₆ alkenyl, provided that R¹³ and R¹⁴ do not simultaneously represent a hydroxyl group;
a compound of formula (IV) 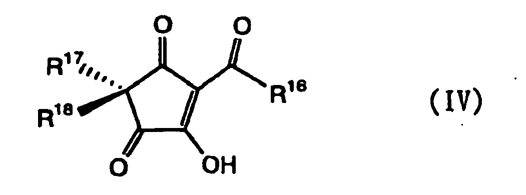 wherein R¹⁶, R¹⁷ and R¹⁶ represent a hydrogen atom, C₁₋₆ alkyl or C₂₋₆ alkenyl; or
a compound of formula (V) 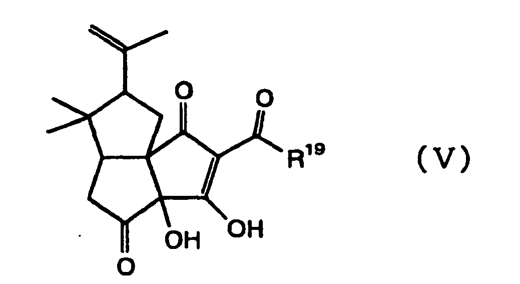 wherein R¹⁹ represents C₁₋₆ alkyl or C₂₋₆ alkenyl;
or a pharmaceutically acceptable salt or solvate thereof (referred to as "an active ingredient according to the present invention" hereinafter) ; or an isomerized hop extract as an active ingredient.

A composition according to the present invention is a composition for use in the amelioration of insulin resistance, the improvement of lipid metabolism, the suppression of body weight gain, or the slimming, comprising an active ingredient according to the present invention, or an isomerized hop extract as an active ingredient.

A composition according to the present invention is a composition for activating PPAR, comprising an active ingredient according to the present invention or an isomerized hop extract as an effective compound.

A food according to the present invention is a food for use in the amelioration of insulin resistance, the improvement of lipid metabolism, the suppression of weight gain, or the slimming, comprising an active ingredient according to the present invention or an isomerized hop extract as an active ingredient.

Insulin-resistant diabetes and hyperlipidemia are chronic diseases and their pathophysiology is complicated and associated with lipid metabolism abnormalities and in the circulatory system along with abnormalities in sugar metabolism. Their treatment with drugs often requires long period of time and various problems such as incidence of side effects due to increased dosages and prolonged administration cannot be ignored. An active ingredient of a composition according to the present invention is contained in hops that have been used as a food for many years. Therefore, a composition according to the present invention is advantageous in that it has little side effects and highly safe when taken by a patient over a long period of time.

### BRIEF DESCRIPTION OF THE DRAWINGS

Figure 1 shows the change in the total cholesterol concentration in the blood in Example 1. In the Figure, * represents a significance level of 5% or less (the same hereinafter). The black square represents the group administered with Kettle, and the black triangle represents the control group.
Figure 2 shows the change in the HDL cholesterol concentration in the blood in Example 1. The black square represents the group administered with Kettle, and the black triangle represents the control group.
Figure 3 shows the change in the atherogenic index in Example 1. The black square represents the group administered with Kettle, and the black triangle represents the control group.
Figure 4 shows the change in the triglyceride concentration in the blood in Example 1. The black square represents the group administered with Kettle, and the black triangle represents the control group.
Figure 5 shows the weight of the fat around the kidney per kg body weight in Example 1.
Figure 6 shows the change in the amount of daily intake per mouse in Example 1. The black square represents the group administered with Kettle, and the black triangle represents the control group.
Figure 7 shows the change in body weight of mice in Example 1. The black square represents the group administered with Kettle, and the black triangle represents the control group.
Figure 8 shows the amount of phospholipid (mg) per g liver in Example 1.
Figure 9 shows the amount of cholesterol (mg) per g liver in Example 1.
Figure 10 shows the amount of triglyceride (mg) per g liver in Example 1.
Figure 11 shows the change in the total cholesterol concentration in the blood in Example 2. The significance of difference is not shown.
Figure 12 shows the change in the HDL-cholesterol concentration in the blood in Example 2. The significance of difference is not shown.
Figure 13 shows the change in the atherogenic index in Example 2. The significance of difference is not shown.
Figure 14 shows the distribution of lipoprotein in Example 2. Plasma samples from mice in the control group and the group administered with the water soluble extract were analyzed by gel filtration. A specific increase in the HDL fraction by the water soluble extract is shown.
Figure 15 shows the change in the amount of daily intake per mouse in Example 2.
Figure 16 shows the change in body weight of mice in Example 2.
Figure 17 shows the change in the amount of daily intake per mouse in Example 3.
Figure 18 shows the change in body weight of mice in Example 3.
Figure 19 shows the total cholesterol concentration in the blood upon dissection in Example 3.
Figure 20 shows the HDL cholesterol concentration in the blood upon dissection in Example 3.
Figure 21 shows the atherogenic index upon dissection in Example 3.
Figure 22 shows the amount of cholesterol (mg) per g liver in Example 3.
Figure 23 shows the amount of triglyceride (mg) per g liver in Example 3.
Figure 24 shows the amount of phospholipid (mg) per g liver in Example 3.
Figure 25 shows the weight of the fat around organs (around the epididymis and around the kidney) per kg body weight in Example 3.
Figure 26 shows the total cholesterol concentration in the blood upon dissection in Example 4.
Figure 27 shows the HDL cholesterol concentration in the blood upon dissection in Example 4.
Figure 28 shows the atherogenic index upon dissection in Example 4.
Figure 29 shows the amount of cholesterol (mg) per g liver in Example 4.
Figure 30 shows the amount of triglyceride (mg) per g liver in Example 4.
Figure 31 shows the amount of phospholipid (mg) per g liver in Example 4.
Figure 32 shows the amount of expression of individual genes relative to the expression of the acidic ribosomal protein 36B4 gene in Example 5.
Figure 33 shows the amount of water intake of mice per day in Example 6.
Figure 34 shows the blood sugar level on week 5 under non-fasting conditions in Example 6.
Figure 35 shows the blood sugar level on week 4 under fasting conditions in Example 6.
Figure 36 shows the triglyceride concentration in the blood on week 2 and week 4 under fasting conditions and on week 6 (upon dissection) under non-fasting conditions in Example 6.
Figure 37 shows the free fatty acid concentration in the blood on week 2 and week 4 under fasting conditions and on week 6 (upon dissection) under non-fasting conditions in Example 6.
Figure 38 shows the amount of expression of the resistin gene relative to the expression of the acidic ribosomal protein 36B4 gene upon dissection in the fat around the epididymis in Example 6.
Figure 39 shows the result of the glucose tolerance test in Example 7.
Figure 40 shows the result of the insulin sensitivity test in Example 7.
Figure 41 shows the change in body weight gain in Example 8.
Figure 42 shows the change in diet intake per day in Example 8.
Figure 43 shows the result of the glucose tolerance test in Example 8.
Figure 44 shows the PPARγ activity of humulones and isohumulones in Example 9.
Figure 45 shows the PPARγ activity of tetrahydroisohumulone in Example 9.
Figure 46 shows the PPARγ activity of the hop extract, humulones and isohumulones in Example 10.
Figure 47 shows the PPARα activity of the water soluble hop extract in Example 11.
Figure 48 shows the blood triglyceride concentration (mg/dl) in Example 13.
Figure 49 shows the amount of cholesterol per g liver (mg/g) in Example 13.
Figure 50 shows the amount of triglyceride per g liver (mg/g) in Example 13.
Figure 51 shows the amount of phospholipid per g liver (mg/g) in Example 13.
Figure 52 shows the change in body weight in Example 13. The diamond shape represents the control group (group C), the square represents the group administered with the water soluble extract (group W), and the triangle represents the group administered with isohumulones (group IH).
Figure 53 shows the amount of the body weight gain per calorie (g/kcal) in Example 13.
Figure 54 shows the amount of cholesterol per g liver (mg/g) in Example 14.
Figure 55 shows the amount of triglyceride per g liver (mg/g) in Example 14.
Figure 56 shows the amount of phospholipid per g liver (mg/g) in Example 14.
Figure 57 shows the change in body weight in Example 14. The diamond shape represents the control group (group C) and the square represents the group administered with lupulone (group L).
Figure 58 shows the amount of body weight gain per calorie (g/kcal) in Example 14.
Figure 59 shows the change in the blood sugar level upon OGTT in the experimental group administered with the water soluble hop extract in Example 15. The diamond shape represents the control group (group C), the square represents the group administered with the water soluble extract at 100 mg/kg/day (group W 100), and the triangle represents the group administered with the water soluble extract at 330 mg/kg/day (group W 330).
Figure 60 shows the change in the insulin concentration in the blood upon CGTT in the experimental group administered with the water soluble hop extract in Example 15. The diamond shape represents the control group (group C), the square represents the group administered with the water soluble extract at 100 mg/kg/day (group W 100), and the triangle represents the group administered with the water soluble extract at 330 mg/kg/day (group W 330).
Figure 61 shows the change in the blood sugar level upon OGTT in the experimental group administered with the purified isocohumulone in Example 15. The diamond shape represents the control group (group C), the square represents the group administered with the purified isocohumulone at 10 mg/kg/day (group IH 10), and the triangle represents the group administered with the purified isocohumulone at 30 mg/kg/day (group IH 30).
Figure 62 shows the change in the insulin concentration in the blood upon OGTT in the experimental group administered with the purified isocohumulone in Example 15. The diamond shape represents the control group (group C), the square represents the group administered with the purified isocohumulone at 10 mg/kg/day (group IH 10), and the triangle represents the group administered with the purified isocohumulone at 30 mg/kg/day (group IH 30).
Figure 63 shows the area (%) of atherosclerotic lesions in the thoracic aorta analyzed in Example 16.
Figure 64 shows the area (%) of atherosclerotic lesions in the abdominal aorta analyzed in Example 16.
Figure 65 shows the degree of hypertrophy of the intima of the aortic arch analyzed in Example 16.
Figure 66 shows the degree of hypertrophy of the intima of the aortic valve analyzed in Example 16.
Figure 67 shows the body weight (g) upon dissection measured in Example 16.
Figure 68 shows the weight (g) of intraperitoneal fat upon dissection measured in Example 16.
Figure 69 shows the hepatic triglyceride content (mg/g) upon dissection analyzed in Example 16.
Figure 70 shows the amount of plasma homocysteine (nM/L) upon dissection analyzed in Example 16.
Figure 71 shows the amount of PGE2 production in the large intestine analyzed in Example 17.

### DETAILED DESCRIPTION OF THE INVENTION

### Active ingredients and production methods

The term "C₁₋₆ alkyl" as used herein means a straight or branched chain alkyl group having 1 to 6 carbon atoms. Examples of C₁₋₆ alkyl include methyl, ethyl, propyl, isopropyl, butyl, isobutyl, secondary butyl, tertiary butyl, pentyl, isopentyl, neopentyl, secondary pentyl, and tertiary pentyl. C₁₋₆ alkyl can preferably be C₃₋₅ alkyl.

The term "C₂₋₆ alkenyl" as used herein means a straight or branched chain alkenyl group having 2 to 6 carbon atoms. Examples of C₂₋₆ alkenyl include allyl, butenyl, pentenyl, hexenyl, 3-methyl-1-butene, 3-methyl-2-butene, and 3-methyl-3-butene. C₂₋₆ alkenyl can preferably be C₃₋₅ alkenyl.

R⁵ preferably represents isobutyl, isopropyl, 1-methyl-propyl, ethyl, or isopentyl.

R⁶ preferably represents a hydrogen atom, 3-methyl-2-butene, or isopentyl.

R⁷ preferably represents a hydrogen atom or 3-methyl-2-butene.

R⁸ preferably represents a hydrogen atom, a hydroxyl group, -C(=O)CH₂CH=C(CH₃)₂. -CH(OH)-(CH₂)₂-CH(CH₃)₂, -C(=O)-(CH₂)₂-CH(CH₃)₂, -C (=O) -CH=CH-CH (CH₃)₂, or -CH (OH)-CH₂CH=(CH₃)₂.

R⁹ preferably represents a hydrogen atom, a hydroxyl group, -C(=O)CH₂CH=C(CH₃)₂, -CH(OH)-(CH₂)₂-CH(CH₃)₂, -C(O=)-(CH₂) ₂-CH (CH₃)₂, -C (=O) -CH=CH-CH (CH₃)₂, or -CH (OH)-CH₂CH=C(CH₃)₂.

R¹¹ preferably represents isobutyl, isopropyl, 1-methyl-propyl, ethyl, or isopentyl.

R¹² preferably represents 3-methyl-2-butene.

R¹³ preferably represents a hydroxyl group or -C(=O)-CH=CHCH(CH₃)₂.

R¹⁴ preferably represents a hydroxyl group or -C(=O)-CH=CHCH(CH₃)₂.

R¹⁶ preferably represents isobutyl, isopropyl, 1-methyl-propyl, ethyl, or isopentyl.

R¹⁷ preferably represents 3-methyl-2-butene.

R¹⁸ preferably represents 3-methyl-2-butene.

R¹⁹ preferably represents isobutyl, isopropyl, 1-methyl-propyl, ethyl, or isopentyl.

The compound of formula (II), formula (III), formula (IV), and formula (V), which are one of the effective compounds according to the present invention, represent isohumulones.

Examples of the isohumulones include
cis- or trans-isohumulone,
cis- or trans-isoadhumulone,
cis- or trans-isocohumulone,
cis- or trans-isoposthumulone,
cis- or trans-isoprehumulone,
cis- or trans-tetrahydroisohumulone,
cis- or trans-tetrahydroisoadhumulone,
cis- or trans-tetrahydroisocohumulone,
cis- or trans-tetrahydroisoposthumulone,
cis- or trans-tetrahydroisoprehumulone,
cis- or trans-alloisohumulone,
cis- or trans-alloisoadhumulone,
cis- or trans-alloisocohumulone,
cis- or trans-alloisoposthumulone,
cis- or trans-alloisoprehumulone,
cis- or trans-paraisohumulone,
cis- or trans-paraisoadhumulone,
cis- or trans-paraisocohumulone,
cis- or trans-paraisoposthumulone,
cis- or trans-paraisoprehumulone,
cis- or trans-humulinic acid,
cis- or trans-adhumulinic acid,
cis- or trans-cohumulinic acid,
cis- or trans-posthumulinic acid,
cis- or trans-prehumulinic acid,
cis- or trans-hexahydroisohumulone,
cis- or trans-hexahydroisoadhumulone,
cis- or trans-hexahydroisocohumulone,
cis- or trans-hexahydroisoposthumulone,
cis- or trans-hexahydroisoprehumulone,
cis- or trans-antiisohumulone,
cis- or trans-antiisoadhumulone,
cis- or trans-antiisocohumilone,
cis- or trans-antiisoposthumulone,
cis- or trans-antiisoprehumulone,
hulupone,
adhulupone,
cohulupone,
posthulupone,
prehulupone,
tricyclodehydroisohumulone,
tricyclodehydroisoadhumulone,
tricyclodehydroisocohumulone,
tricyclodehydroisoposthumulone, and
tricyclodehydroisoprehumulone.

Examples of preferred compounds of formula (II) include
a compound wherein R⁵ represents isobutyl, R⁶ represents 3-methyl-2-butene, R⁷ represents a hydrogen atom, R⁸ represents a hydroxyl group, and R⁹ represents - C(=O)CH₂CH=C(CH₃)₂ (cis-isohumulone) ;
a compound wherein R⁵ represents isobutyl, R⁶ represents 3-methyl-2-butene, R⁷ represents a hydrogen atom, R⁸ represents -C(=O)CH₂CH=C(CH₃)₂, and R⁹ represents a hydroxyl group (trans-isohumulone);
a compound wherein R⁵ represents isopropyl, R⁶ represents 3-methyl-2-butene, R⁷ represents a hydrogen atom, R⁸ represents a hydroxyl group, and R⁹ represents - C(=O)CH₂CH=C(CH₃)₂ (cis-isocohumulone);
a compound wherein R⁵ represents isopropyl, R⁶ represents 3-methyl-2-butene, R⁷ represents a hydrogen atom, R⁸ represents -C(=O)CH₂CH=C(CH₃)₂, and R⁹ represents a hydroxyl group (trans-isocohumulone);
a compound wherein R⁵ represents 1-methyl-propyl, R⁶ represents 3-methyl-2-butene, R⁷ represents a hydrogen atom, R⁸ represents a hydroxyl group, and R⁹ represents - C(=O)CH₂CH=C(CH₃)₂ (cis-isoadhumulone) ;
a compound wherein R⁵ represents 1-methyl-propyl, R⁶ represents 3-methyl-2-butene, R⁷ represents a hydrogen atom, R⁸ represents -C(=O)CH₂CH=C(CH₃)₂, and R⁹ represents a hydroxyl group (trans-isoadhumulone);
a compound wherein R⁵ represents ethyl, R⁶ represents 3-methyl-2-butene, R⁷ represents a hydrogen atom, R⁸ represents a hydroxyl group, and R⁹ represents - C(=O)CH₂CH=C(CH₃)₂ (cis-isoposthumulone);
a compound wherein R⁵ represents ethyl, R⁶ represents 3-methyl-2-butene, R⁷ represents a hydrogen atom, R⁸ represents -C(=O)CH₂CH=C(CH₃)₂, and R⁹ represents a hydroxyl group (trans-isoposthumulone);
a compound wherein R⁵ represents isopentyl, R⁶ represents 3-methyl-2-butene, R⁷ represents a hydrogen atom, R⁸ represents a hydroxyl group, and R⁹ represents - C(=O)CH₂CH=C(CH₃)₂ (cis-isoprehumulone) ;
a compound wherein R⁵ represents isopentyl, R⁶ represents 3-methyl-2-butene, R⁷ represents a hydrogen atom, R⁸ represents -C(=O)CH₂CH=C(CH₃)₂, and R⁹ represents a hydroxyl group (trans-isoprehumulone);
a compound wherein R⁵ represents isobutyl, R⁶ represents isopentyl, R⁷ represents a hydrogen atom, R⁸ represents a hydroxyl group, and R⁹ represents - C(=O)CH₂CH₂CH(CH₃)₂ (isohexanoyl group) (cis-tetrahydroisohumulone);
a compound wherein R⁵ represents isobutyl, R⁶ represents isopentyl, R⁷ represents a hydrogen atom, R⁸ represents -C(=O)CH₂CH₂CH(CH₃)₂ (isohexanoyl group), and R⁹ represents a hydroxyl group (trans-tetrahydroisohumulone);
a compound wherein R⁵ represents isopropyl, R⁶ represents isopentyl, R⁷ represents a hydrogen atom, R⁸ represents a hydroxyl group, and R⁹ represents - C(=O)CH₂CH₂CH(CH₃)₂ (isohexanoyl group) (cis-tetrahydroisocohumulone);
a compound wherein R⁵ represents isopropyl, R⁶ represents isopentyl, R⁷ represents a hydrogen atom, R⁸ represents -C(=O)CH₂CH₂CH(CH₃)₂ (isohexanoyl group), and R⁹ represents a hydroxyl group (trans-tetrahydroisocohumulone);
a compound wherein R⁵ represents 1-methyl-propyl, R⁶ represents isopentyl, R⁷ represents a hydrogen atom, R⁸ represents a hydroxyl group, and R⁹ represents - C(=O)CH₂CH₂CH(CH₃)₂ (isohexanoyl group) (cis-tetrahydroisoadhumulone) ;
a compound wherein R⁵ represents 1-methyl-propyl, R⁶ represents isopentyl, R⁷ represents a hydrogen atom, R⁸ represents -C(=O)CH₂CH₂CH(CH₃)₂ (isohexanoyl group), and R⁹ represents a hydroxyl group (trans-tetrahydroisoadhumulone);
a compound herein. R⁵ represents ethyl, R⁶ represents isopentyl, R⁷ represents a hydrogen atom, R⁸ represents a hydroxyl group, and R⁹ represents -C (=O) CH₂CH₂CH (CH₃)₂ (isohexanoyl group) (cis-tetrahydroisoposthumulone);
a compound wherein R⁵ represents ethyl, R⁶ represents isopentyl, R⁷ represents a hydrogen atom, R⁸ represents-C(=O)CH₂CH₂CH(CH₃)₂ (isohexanoyl group), and R⁹ represents a hydroxyl group (trans-tetrahydroisoposthumulone);
a compound wherein R⁵ represents isopentyl, R⁹ represents isopentyl, R⁷ represents a hydrogen atom, R⁸ represents a hydroxyl group, and R⁹ represents - C(=O)CH₂CH₂CH(CH₃)₂ (isohexanoyl group) (cis-tetrahydroisoprehumulone);
a compound wherein R⁵ represents isopentyl, R⁶ represents isopentyl, R⁷ represents a hydrogen atom, R⁸ represents -C(=O)CH₂CH₂CH(CH₃)₂ (isohexanoyl group), and R⁹ represents a hydroxyl group (trans-tetrahydroisoprehumulone);
a compound wherein R⁵ represents isobutyl, R⁶ represents 3-methyl-2-butene, R⁷ represents a hydrogen atom, R⁸ represents a hydroxyl group, and R⁹ represents - C(=O)CH=CHCH(CH₃)₂ (cis-alloisohumulone) ;
a compound wherein R⁵ represents isobutyl, R⁶ represents 3-methyl-2-butene, R⁷ represents a hydrogen atom, R⁸ represents -C(=O)CH=CHCH(CH₃)₂, and R⁹ represents a hydroxyl group (trans-alloisohumulone);
a compound wherein R⁵ represents isopropyl, R⁶ represents 3-methyl-2-butene, R⁷ represents a hydrogen atom, R⁸ represents a hydroxyl group, and R⁹ represents - C(O)CH=CHCH(CH₃)₂ (cis-alloisocohumulone) ;
a compound wherein R⁵ represents isopropyl, R⁶ represents 3-methyl-2-butene, R⁷ represents a hydrogen atom, R⁸ represents -C(=O)CH=CHCH(CH₃)₂, and R⁹ represents a hydroxyl group (trans-alloisocohumulone);
a compound wherein R⁵ represents 1-methyl-propyl, R⁶ represents 3-methyl-2-butene, R⁷ represents a hydrogen atom, R⁸ represents a hydroxyl group, and R⁹ represents-C(=O)CH=CHCH(CH₃)₂ (cis-alloisoadhumulone);
a compound wherein R⁵ represents 1-methyl-propyl, R⁶ represents 3-methyl-2-butene, R⁷ represents a hydrogen atom, R⁸ represents -C(=O)CH=CHCH(CH₃)₂, and R⁹ represents a hydroxyl group (trans-alloisoadhumulone);
a compound wherein R⁵ represents ethyl, R⁶ represents 3-methyl-2-butene, R⁷ represents a hydrogen atom, R⁸ represents a hydroxyl group, and R⁹ represents - C(=O)CH=CHCH(CH₃)₂ (cis-alloisoposthumulone);
a compound wherein R⁵ represents ethyl, R⁶ represents 3-methyl-2-butene, R⁷ represents a hydrogen atom, R⁸ represents -C(=O)CH=CHCH(CH₃)₂, and R⁹ represents a hydroxyl group (trans-alloisoposthumulone);
a compound wherein R⁵ represents isopentyl, R⁶ represents 3-methyl-2-butene, R⁷ represents a hydrogen atom, R⁸ represents a hydroxyl group, and R⁹ represents - C(=O)CH=CHCH(CH₃)₂ (cis-alloisoprehumulone) ;
a compound wherein R⁵ represents isopentyl, R⁶ represents 3-methyl-2-butene, R⁷ represents a hydrogen atom, R⁸ represents -C(=O)CH=CHCH(CH₃)₂, and R⁹ represents a hydroxyl group (trans-alloisoprehumulone);
a compound wherein R⁵ represents isobutyl, R⁶ represents 3-methyl-2-butene, R⁷ represents a hydrogen atom, R⁸ represents a hydroxyl group, and R⁹ represents -CH(-OH)CH₂CH=C(CH₃)₂ (cis-paraisohumulone);
a compound wherein R⁵ represents isobutyl, R⁶ represents 3-methyl-2-butene, R⁷ represents a hydrogen atom, R⁸ represents -CH(-OH)CH₂CH=C(CH₃)₂, and R⁹ represents a hydroxyl group (trans-paraisohumulone);
a compound wherein R⁵ represents isopropyl, R⁶ represents 3-methyl-2-butene, R⁷ represents a hydrogen atom, R⁸ represents a hydroxyl group, and R⁹ represents -CH (-OH)CH₂CH=C(CH₃)₂ (cis-paraisocohumulone) ;
a compound wherein R⁵ represents isopropyl, R⁶ represents 3-methyl-2-butene, R⁷ represents a hydrogen atom, R⁸ represents -CH(-OH)CH₂CH=C(CH₃)₂, and R⁹ represents a hydroxyl group (trans-paraisocohumulone);
a compound wherein R⁵ represents 1-methyl-propyl, R⁶ represents 3-methyl-2-butene, R⁷ represents a hydrogen atom, R⁸ represents a hydroxyl group, and R⁹ represents -CH(-OH)CH₂CH=C(CH₃)₂ (cis-paraisoadhumulone) ;
a compound wherein R⁵ represents 1-methyl-propyl, R⁶ represents 3-methyl-2-butene, R⁷ represents a hydrogen atom, R⁸ represents -CH(-OH)CH₂CH=(CH₃)₂, and R⁹ represents a hydroxyl group (trans-paraisoadhumulone);
a compound wherein R⁵ represents ethyl, R⁶ represents 3-methyl-2-butene, R⁷ represents a hydrogen atom, R⁸ represents a hydroxyl group, and R⁹ represents -CH(-OH)CH₂CH=(CH₃)₂ (cis-paraisoposthumulone) ;
a compound wherein R⁵ represents ethyl, R⁶ represents 3-methyl-2-butene, R⁷ represents a hydrogen atom, R⁸ represents -CH(-OH)CH₂CH=C(CH₃)₂, and R⁹ represents a hydroxyl group (trans-paraisoposthumulone);
a compound wherein R⁵ represents isopentyl, R⁶ represents 3-methyl-2-butene, R⁷ represents a hydrogen atom, R⁸ represents a hydroxyl group, and R⁹ represents -CH(-OH)CH₂CH=C(CH₃)₂ (cis-paraisoprehumulone) ;
a compound wherein R⁵ represents isopentyl, R⁶ represents 3-methyl-2-butene, R⁷ represents a hydrogen atom, R⁸ represents -CH(-OH)CH₂CH=C(CH₃)₂, and R⁹ represents a hydroxyl group (trans-paraisoprehumulone);
a compound wherein R⁵ represents isobutyl, R⁶ represents 3-methyl-2-butene, R⁷ represents a hydrogen atom, R⁸ represents a hydroxyl group, and R⁹ represents a hydrogen atom (cis-humulinic acid);
a compound wherein R⁵ represents isobutyl, R⁶ represents 3-methyl-2-butene, R⁷ represents a hydrogen atom, R⁸ represents a hydrogen atom, and R⁹ represents a hydroxyl group (trans-humulinic acid);
a compound wherein R⁵ represents isopropyl, R⁶ represents 3-methyl-2-butene, R⁷ represents a hydrogen atom, R⁸ represents a hydroxyl group, and R⁹ represents a hydrogen atom (cis-cohumulinic acid) ;
a compound wherein R⁵ represents isopropyl, R⁶ represents 3-methyl-2-butene, R⁷ represents a hydrogen atom, R⁸ represents a hydrogen atom, and R⁹ represents a hydroxyl group (trans-cohumulinic acid);
a compound wherein R⁵ represents 1-methyl-propyl, R⁶ represents 3-methyl-2-butene, R⁷ represents a hydrogen atom, R⁸ represents a hydroxyl group, and R⁹ represents a hydrogen atom (cis-adhumulinic acid);
a compound wherein R⁵ represents 1-methyl-propyl, R⁶ represents 3-methyl-2-butene, R⁷ represents a hydrogen atom, R⁸ represents a hydrogen atom, and R⁹ represents a hydroxyl group (trans-adhumulinic acid);
a compound wherein R⁵ represents ethyl, R⁶ represents 3-methyl-2-butene, R⁷ represents a hydrogen atom, R⁸ represents a hydroxyl group, and R⁹ represents a hydrogen atom (cis-posthumulinic acid) ;
a compound wherein R⁵ represents ethyl, R⁶ represents 3-methyl-2-butene, R⁷ represents a hydrogen atom, R⁸ represents a hydrogen atom, and R⁹ represents a hydroxyl group (trans-posthumulinic acid);
a compound wherein R⁵ represents isopentyl, R⁶ represents 3-methyl-2-butene, R⁷ represents a hydrogen atom, R⁸ represents a hydroxyl group, and R⁹ represents a hydrogen atom (cis-prehumulinic acid);
a compound wherein R⁵ represents isopentyl, R⁶ represents 3-methyl-2-butene, R⁷ represents a hydrogen atom, R⁸ represents a hydrogen atom, and R⁹ represents a hydroxyl group (trans- prehumulinic acid);
a compound wherein R⁵ represents isobutyl, R⁶ represents isopentyl, R⁷ represents a hydrogen atom, R⁸ represents a hydroxyl group, and R⁹ represents -CH(-OH)CH₂CH₂CH(CH₃)₂ (cis-hexahydroisohumulone);
a compound wherein R⁵ represents isobutyl, R⁶ represents isopentyl, R⁷ represents a hydrogen atom, R⁸ represents -CH(-OH)CH₂CH₂CH(CH₃)₂, and R⁹ represents a hydroxyl group (trans-hexahydroisohumulone);
a compound wherein R⁵ represents isopropyl, R⁶ represents isopentyl, R⁷ represents a hydrogen atom, R⁸ represents a hydroxyl group, and R⁹ represents -CH(-OH) CH₂CH₂CH (CH₃)₂ (cis-hexahydroisocohumulone) ;
a compound wherein R⁵ represents isopropyl, R⁶ represents isopentyl, R⁷ represents a hydrogen atom, R⁸ represents -CH(-OH)CH₂CH₂CH(CH₃)₂, and R⁹ represents a hydroxyl group (trans-hexahydroisocohumulone);
a compound wherein R⁵ represents 1-methyl-propyl, R⁶ represents isopentyl, R⁷ represents a hydrogen atom, R⁸ represents a hydroxyl group, and R⁹ represents -CH(-OH)CH₂CH₂CH(CH₃)₂ (cis-hexahydroisoadhumulone);
a compound wherein R⁵ represents 1-methyl-propyl, R⁶ represents isopentyl, R⁷ represents a hydrogen atom, R⁸ represents -CH (-OH) CH₂CH₂CH (CH₃)₂, and R⁹ represents a hydroxyl group (trans-hexahydroisoadhumulone);
a compound wherein R⁵ represents ethyl, R⁶ represents isopentyl, R⁷ represents a hydrogen atom, R⁸ represents a hydroxyl group, and R⁹ represents -CH(-OH) CH₂CH₂CH (CH₃)₂ (cis-hexahydroisoposthumulone);
a compound wherein R⁵ represents ethyl, R⁶ represents isopentyl, R⁷ represents a hydrogen atom, R⁸ represents -CH(-OH)CH₂CH₂CH(CH₃)₂, and R⁹ represents a hydroxyl group (trans-hexahydroisoposthumulone);
a compound wherein R⁵ represents isopentyl, R⁶ represents isopentyl, R⁷ represents a hydrogen atom, R⁸ represents a hydroxyl group, and R⁹ represents -CH(-OH) CH₂CH₂CH (CH₃)₂ (cis-hexahydroisoprehumulone); and
a compound wherein R⁵ represents isopentyl, R⁶ represents isopentyl, R⁷ represents a hydrogen atom, R⁸ represents -CH (-OH) CH₂CH₂CH (CH₃) ₂, and R⁹ represents a hydroxyl group (trans-hexahydroisoprehumulone).

Examples of preferred compounds of formula (III) include
a compound wherein R¹¹ represents isobutyl, R¹² represents 3-methyl-2-butene, R¹³ represents - C(=O)CH₂CH=C(CH₃)₂, and R¹⁴ represents a hydroxyl group (cis-antiisohumulone);
a compound wherein R¹¹ represents isopropyl, R¹² represents 3-methyl-2-butene, R¹³ represents-C(=C)CH₂CH=C-(CH₃)₂, and R¹⁴ represents a hydroxyl group (cis-antiisocohumulone) ;
a compound wherein R¹¹ represents 1-methyl-propyl, R¹² represents 3-methyl-2-butene, R¹³ represents - C (=O) CH₂CH=C (CH₃) ₂, and R¹⁴ represents a hydroxyl group (cis-antiisoadhumulone);
a compound wherein R¹¹ represents ethyl, R¹² represents 3-methyl-2-butene, R¹³ represents -C (=O) CH₂CH=C (CH₃) ₂, and R¹⁴ represents a hydroxyl group (cis-antiisoposthumulone);
a compound wherein R¹¹ represents isopentyl, R¹² represents 3-methyl-2-butene, R¹³ represents - C(=O) CH₂CH=C (CH₃)₂, and R¹⁴ represents a hydroxyl group (cis-antiisoprehumulone);
a compound wherein R¹¹ represents isobutyl, R¹² represents 3-methyl-2-butene, R¹³ represents a hydroxyl group, and R¹⁴ represents -C (=O) CH₂CH=C (CH₃)₂ (trans-antiisohumulone);
a compound wherein R¹¹ represents isopropyl, R¹² represents 3-methyl-2-butene, R¹³ represents a hydroxyl group, and R¹⁴ represents -C (=O) CH₂CH=C (CH₃)₂ (trans-antiisocohumulone);
a compound wherein R¹¹ represents 1-methyl-propyl, R¹² represents 3-methyl-2-butene, R¹³ represents a hydroxyl group, and R¹⁴ represents -C(=O) CH₂CH=C(CH₃)₂ (trans-antiisoadhumulone);
a compound wherein R¹¹ represents ethyl, R¹² represents 3-methyl-2-butene, R¹³ represents a hydroxyl group, and R¹⁴ represents -C(=O)CH₂CH=C(CH₃)₂ (trans-antiisoposthumulone) ; and
a compound wherein R¹¹ represents isopentyl, R¹² represents 3-methyl-2-butene, R¹³ represents a hydroxyl group, and R¹⁴ represents -C(=O)CH₂CH=C(CH₃)₂ (trans-antiisoprehumulone).

Examples of preferred compounds of formula (IV) include
a compound wherein R¹⁶ represents isobutyl, R¹⁷ represents 3-methyl-2-butene, and R¹³ represents 3-methyl-2-butene (hulupone);
a compound wherein R¹⁶ represents isopropyl, R¹⁷ represents 3-methyl-2-butene, and R¹³ represents 3-ethyl-2-butene (cohulupone);
a compound wherein R¹⁶ represents 1-methyl-propyl, R¹⁷ represents 3-methyl-2-butene, and R¹³ represents 3-methyl-2-butene (adhulupone);
a compound wherein R¹⁶ represents ethyl, R¹⁷ represents 3-methyl-2-butene, and R¹³ represents 3-methyl-2-butene (posthulupone); and
a compound wherein R¹⁶ represents isopentyl, R¹⁷ represents 3-methyl-2-butene, and R¹³ represents 3-methyl-2-butene (prehulupone).

Examples of preferred compounds of formula (V) include
a compound wherein R¹⁹ is isobutyl (tricyclodehydroisohumulone),
a compound wherein R¹⁹ is isopropyl (tricyclodehydroisocohumulone),
a compound wherein R¹⁹ is 1-methyl-propyl (tricyclodehydroisoadhumulone),
a compounds wherein R¹⁹ is ethyl (tricyclodehydroisoposthumulone), and
a compound wherein R¹⁹ is isopentyl (tricyclodehydroisoprehumulone).

The compounds of formula (II), formula (III), formula (IV), and formula (V) can be pharmaceutically acceptable salts, such as acid addition salts. Examples of the acid addition salts include salts of inorganic acids such as hydrochloric acid, hydrobromic acid and sulfuric acid; and salts of organic acids such as citric acid, oxalic acid, malic acid, tartaric acid, fumaric acid, maleic acid, methanesulfonic acid, and salicylic acid. Further, compounds having a carboxyl group can be salts with metals such as sodium, potassium, calcium, magnesium and aluminium, or salts with amino acids such as lysine.

The compounds of formula (II), formula (III), formula (IV), and formula (V) can be pharmaceutically acceptable solvates, such as hydrates, alcoholates (for example, methanolates and ethanolates) and etherates.

Cis-trans isomers derived from substituents, alkenyl groups, can be found in the compounds of formula (II), formula (III), formula (IV), and formula (V), and the present invention includes all of these isomers and mixtures thereof.

The active ingredients according to the present invention are commercially available.

The active ingredients according to the present invention can be produced according to known methods; for example, they can be synthesized by the method described in Developments in Food Science 27, CHEMISTRY AND ANALYSIS OF HOP AND BEER BITTER ACIDS, M. Verzele, ELSEVIER. 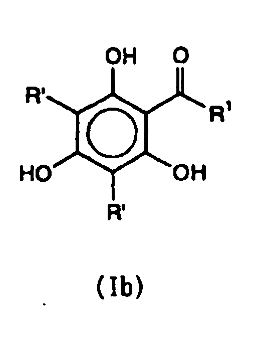 In the formulas above, R¹ and R' is C₁₋₆ alkyl or C₂₋₆ alkenyl.

Humulone, which may be used as a starting material in the preparation of compounds of formula II-V can be obtained by oxidizing the compound of formula (Ib) and adding an alkenyl side chain onto an aromatic carbon. There are various methods for the oxidation reaction. For example, the oxidation can be carried out by reaction with antimony pentachloride at -50°C, followed by hydrolyzation in the presence of silver ion. Further, the oxidation reaction can be carried out with lead acetate in the presence of an acetic acid solution or in the presence of trifluoroacetic acid and hydrogen peroxide. Alternatively, the oxidation reaction can be carried out by reaction with benzoyl peroxide in the presence of alkali catalyst, or by reaction with diphenylseleninic anhydride in the presence of dichloromethane.

The compound of formula (II) can be produced from 2-methyl-2-penten-4-yne. 2-Methyl-2-penten-4-yne can be obtained by a 1,4-elimination reaction of 1-bromo-4-methylpent-1,2-diene in the presence of Cu₂(CN)₂. Further, 2,6-dimethyl-2-hydroxy-5-hepten-3-yne acid can be obtained by adding 2-methyl-2-penten-4-yne thus obtained to ethyl pyruvate for hydrolysis reaction. (COCl)₂ is added to the solution obtained and the resulting Cl salt is added to ethyl 3-oxo-5-methylhexanoate in the presence of a magnesium salt to obtain cyclized 2-(3-methylbutanoyl)-3,4-dihydroxy-4-(4-methyl-3-penten-1-ynyl)-2-cyclopentenone. Isohumulone can be obtained by reacting the obtained compound with 1-bromo-3-methyl-2-butene and hydrating the triple bond.

Cis- or trans-alloisohumulone represented by formula (II) can be obtained using humulone as a starting material. For example, using adhumulone, cohumulone, posthumulone, or prehumulone as a starting material, cis- or trans-alloisoadhumulone, cis- or trans-alloisocohumulone, cis- or trans-alloisoposthumulone, or cis- or trans-alloisoprehumulone can be produced, respectively.
Cis- or trans-alloisohumulone can be produced, for example, by the method of F. Alderweireldt et al. (Bull. Soc. Chim. Belges, 74 (1965) 29) or the method of M. Verzele et al. (J. Inst. Brewing, 71 (1965) 232). Humulone is boiled in a phosphoric acid buffer solution (pH 9.0) for 1. hour. After cooling, the pH is adjusted to 1.0 with hydrochloric acid, extraction is carried out with isooctane and the solvent is evaporated by drying. Then, the isooctane and the aqueous phase, which is a pH 5.5 buffer solution, can be separated using a counter-current distribution method (referred to as CCD method hereinafter) to fractionate cis-alloisohumulone and trans-alloisohumulone.

Cis- or trans-humulinic acid represented by formula (II) can be obtained using humulone as a starting material. For example, using adhumulone, cohumulone, posthumulone, or prehumulone as a starting material, cis- or trans-adhumulinic acid, cis- or trans-cohumulinic acid, cis- or trans-posthumulinic acid, or cis- or trans-prehumulinic acid can be produced, respectively. Cis- or trans-humulinic acid can be produced, for example, by hydrolyzing humulone in a strong alkaline solution (H. Wieland, Ber. 59 (1926) 2352; or J.F. Carson, J. Am. Chem. Soc., 74 (1952) 4615), preferably by adding dropwise 2 N sodium hydroxide in methanol and heating at 67°C for 20 minutes under nitrogen gas. The reaction is stopped with cold 2 N hydrochloric acid and extraction is carried out with chloroform, after which the solvent is evaporated and then the chloroform and the aqueous phase, which is a pH 5.1 buffer solution, can be separated using the CCD method for fractionation.

Cis- or trans-tetrahydroisohumulone represented by formula (II) can be obtained using cis- or trans-isohumulone as a starting material. For example, using cis- or trans-isoadhumulone, cis- or trans-isocohumulone, cis- or trans-isoposthumulone, or cis- or trans-isoprehumulone as a starting material, cis- or trans-tetrahydroisoadhumulone, cis- or trans-tetrahydroisocohumulone, cis- or trans-tetrahydroisoposthumulone, or cis- or trans-tetrahydroisoprehumulone can be produced, respectively. Cis- or trans-tetrahydroisohumulone can be produced, for example, by catalytic hydrogenation of cis- or trans-isohumulone in methanol by the use of palladium on carbon, preferably by evaporating the solvent for drying to solid after the hydrogenation and then recrystalizing in isooctane. Commercially available tetrahydroisohumulones can also be used.

Cis- or trans-hexahydroisohumulone represented by formula (II) can be obtained using cis- or trans-tetrahydroisohumulone as a starting material. For example, using cis- or trans-tetrahydroisoadhumulone, cis- or trans-tetrahydroisocohumulone, cis- or trans-tetrahydroisoposthumulone, or cis- or trans-tetrahydroisoprehumulone as a starting material, cis- or trans-hexahydroisoadhumulone, cis- or trans-hexahydroisocohumulone, cis- or trans-hexahydroisoposthumulone, or cis- or trans-hexahydroisoprehumulone can be produced, respectively. Cis- or trans-hexahydroisohumulone can be produced, for example, by reducing cis- or trans-tetrahydroisohumulone with NaBH₄. Commercially available hexahydroisohumulones can also be used.

The compounds of formula (III), formula (IV), and formula (V) can be obtained by extracting and purifying compounds found in hop corns, hop extracts or isomerized material thereof, and if necessary, further appropriately modifying them, as described below.

Cis- or trans-antiisohumulone represented by formula (III) can be obtained using humulone as a starting material. For example, using adhumulone, cohumulone, posthumulone, or prehumulone as a starting material, cis- or trans-antiisoadhumulone, cis- or trans-antiisocohumulone, cis- or trans-antiisoposthumulone, or cis- or trans-antiisoprehumulone can be produced, respectively. More specifically, cis- or trans-antiisohumulone can be produced by boiling humulone in an aqueous solution at pH 5.4-11.0. The pH is preferably about 11.0 and the reaction time is preferably about 1.5 hours. After boiling, the solution is cooled, acidified with hydrochloric acid and then extracted with isooctane, and after evaporation and drying to solid, ether and the aqueous phase, which is a pH 5.5 buffer solution, are separated using the CCD method to fractionate the cis-antiisohumulone and trans-antiisohumulone.

Hulupone represented by formula (IV) can be produced using lupulone as a starting material. For example, using adlupulone, colupulone, postlupulone, or prelupulone as a starting material, adhulupone, cohulupone, posthulupone, or prehulupone can be produced, respectively. More specifically, hulupone can be produced by oxidizing lupulone (D. Wright, proc. Chem. Soc., 315 (1961); D. Wright, J. Chem. Soc., 1769 (1963)). For example, hulupone can be produced by shaking lupulone in cyclohexane under oxygen, removing the solvent, and then separating light yellow oil by distillation. More preferably, sodium sulfite is added to lupulone in methanol and the admixture is shaken under oxygen gas until gas absorption cannot be observed, after which the solvent is removed, the residue is extracted twice with warmed hexane, the extract is suspended in methanol, the suspension is acidified with 2 N hydrochloric acid and diluted with water, extraction is again carried out with hexane, and then hulupone can be produced by distillation.

An active ingredient according to the present invention can be an isomerized hop extract. A hop extract is found, for example, in hop strobili. Hop extracts or isomerized products thereof can be fractionated using various chromatographic methods (see "The components of Brewing Product," December 10, 1999, published by Brewing Society of Japan; the abovementioned Developments in Food Science 27, CHEMISTRY AND ANALYSIS OF HOP AND BEER BITTER ACIDS; and reference examples below) . Further, a large amount of highly pure humulone, adhumulone and cohumulone can be purified from a supercritical extract of hop strobili (hop extract) using centrifugal partition chromatography (A.C.J. Hermans-Lokkerbol et al., J. Chromatography A664 (1994) pp. 45-53). Further, a pure compound can be obtained by recrystalizing their mixture. For example, a specific complex consisting of 1,2-diaminobenzene and humulones can be formed by adding 1,2-diaminobenzene to the supercritical extract of hop strobili (hop extract). By repeatedly crystallizing this complex, a complex consisting of humulone contained at the highest concentration and 1,2-diaminobanzene can be specifically crystallized. Highly pure humulone can be obtained by dissolving the crystallized compound in methanol and separating 1,2-diaminobenzene using resins such as zeolite (see Colin P. et al., J. Inst. Brew. June-July, 1993, Vol. 99, pp. 347-348). These methods are all described in Developments in Food Science 27, CHEMISTRY AND ANALYSIS OF HOP AND BEER BITTER ACIDS, M. Verzele, ELSEVIER and thus can readily be carried out by anyone skilled in the art.

In a composition according to the present invention, extract derived from hop lupulin can be used as an active ingredient after isomerization. The hop is a perennial plant which belongs to the family Cannabaceae, and hops are its strobili (matured unpollinated female flowers). Hop lupulin is a raw material for beer brewing and is used to impart bitter taste and aroma to the beer. Further, in the beer brewing process, humulones (e.g., cohumulone, adhumulone, posthumulone, and prehumulone) are isomerized to isohumulones (e.g., isocohumulone, isoadhumulone, isoposthumulone, and isoprehumulone) to impart characteristic taste and aroma to the beer.

A hop extract can be prepared by subjecting strobili or pressed product thereof, as it is or after crushing, to an extraction process. The extraction can be carried out, for example, by a method used for the preparation of hop extract for the beer brewing, such as the extraction method using ethanol solvent and the supercritical carbon dioxide extraction method. In particular, the supercritical carbon dioxide extraction is characterized in that the resulting product contains a low concentration of polyphenol component and bitter component and essential oil component are highly concentrated. Further, hop extraction can be carried out using other generally used methods, including a method in which hop strobili, crushed products thereof, or the like are submersed in a cold or warmed solvent; a method in which extraction is carried out with heating and stirring and then the resulting extract is obtained by filtration; and a percolation method. After removing solids by filtration or centrifugation if necessary, the resulting extract can be used as it is or after removing the solvent by distillation and partially concentrating or drying, depending on the mode of use. Further, after concentrating or drying, the extract can be washed and purified with an insoluble solvent or further dissolved and suspended in an appropriate solvent for use. Further in the present invention, for example, the solvent extract obtained as described above can be dried using general means such as drying under the reduced pressure and freeze drying to obtain a dried hop extract for use.

Examples of solvents to be used for the abovementioned extraction include water; lower alcohols having 1-4 carbon atoms, such as methanol, ethanol, propanol and butanol; lower alkyl esters such as ethyl acetate ester; glycols such as ethylene glycol, butylene glycol, propylene glycol, and glycerin; other polar solvents such as ethyl ether, acetone, and acetic acid; hydrocarbons such as benzene and hexane; non-polar solvent such as ethers, e.g., ethyl ethers and petroleum ethers, or known organic solvents. These solvents can be used alone or in combination of two or more kinds.

Further, if necessary, insolubles can be removed by filtration, concentration can be carried out, for example, under the reduced pressure, or the solvent can be dried to solid. Further, preferably, crushed strobili are subjected to the supercritical carbon dioxide extraction or the liquid carbon dioxide gas extraction. It is also preferable to isomerize these crude extracts by heating in the presence of alkali or magnesium oxide. By the isomerization, humulones are converted into isohumulones. The extracts thus obtained can be used as they are for pharmaceutical preparations; however, it is also preferable to use a fraction containing active ingredients at higher concentrations. Hop extracts extracted by various methods and isomerized extracts are commercially available as a beer additive. Examples of the usable products include isomerised form of a hop extract in which humulones and lupulones are primarily extracted from crushed hop strobili using the supercritical carbon dioxide extraction method (e.g., CO2 Pure Resin Extract (Hopsteiner)), an isomerized carbon dioxide extract of crushed hop strobili (e.g., Isomerized Kettle Extract (SS. Steiner) mainly consisting of isohumulones and lupulones), and a water soluble extract in which carbon dioxide extract of crushed hop strobili is isomerized and then converted into a potassium salt to obtain a low viscous fluid (e.g., ISOHOPCO2N (English Hop Products) primarily consisting of isohumulones).

Further, it should be understood that these extracts can be further concentrated to fractions containing highly concentrated active ingredients by using the abovementioned methods or the like.

### Use

Active ingredients according the present invention have PPARα agonist activity and PPARγ agonist activity (see Examples 9, 10 and 11).

It is known that PPARα is deeply involved in lipid metabolism and that a fibrate drug that is a synthetic ligand of PPARα accelerates intravascular lipoprotein lipase activity and hepatic β-oxidation, and acts on the activation of fatty acid binding protein in the liver to enhance fatty acid flow into the liver and suppress the hepatic VLDL production, which results in lowering the VLDL level in the blood ("Transfiguring Lifestyle Diseases - Diabetes, Hyperlipidemia, Hypertension, and Obesity," published by Medical Review, May 25, 2000).

Further, a fibrate drug that is a PPARα ligand is considered to ameliorate insulin resistance (Guerre-Millo M. et al., J. B. C., 275:16638-16642, 2000). The PPARα ligand probably enhances fatty acid oxidation in the liver and other tissues to reduce fat toxicity, ameliorates the efficiency of glucose metabolism, and removes insulin resistance.

PPARγ is shown to be a master regulator to control adipocyte differentiation (Cell 79:1147-1156, 1994). Therefore, a PPARγ agonist promotes the adipocyte differentiation. Probable mechanisms of such amelioration in insulin resistance by this PPARγ activation are explained as follows. Adipocytes having normal functions generated by PPARγ activation increase their capability in treating sugar and free fatty acid, which results in the reduction of the sugar and free fatty acid levels in the blood, the reduction of the muscular free fatty acid level and the amelioration of insulin resistance. Further, adipocytes excrete important physiologically active mediators which deteriorate insulin resistance, such as TNFα and resistin; adipocyte differentiation by the PPARγ activation is revealed to reduce the secretion of these mediators. Further, agonistic action to PPARγ expressed in a small amount in the muscle and liver is also probable.

An extract containing an active ingredient according to the present invention suppresses, at the gene level, the expression of resistin which is considered to be increasingly expressed in the case of non-insulin independent diabetes and take part in the incidence of insulin resistance (see Example 6). The correlation between resistin and the incidence of insulin resistance is reported in Peraldi P., et al., Mol. Cell Biochem., 183, 169-175, 1998; Steppan C.M. et al., Nature, 409, 307-312, 2001.

It is known that insulin resistance causes hyperlipidemia. A mechanism associated with the hyperlipidemia is considered as follows. When insulin resistance is generated in skeletal muscle and adipose tissue, an excessive amount of insulin is secreted from the pancreas to normalize impaired glucose tolerance accompanied by the insulin resistance so as to maintain the blood sugar homeostasis. Hyperinsulinemia thus induced causes an increase in blood pressure and lipid metabolism abnormalities. Insulin normally suppresses lipolysis in adipose tissue; however this suppressing ability declines in the state of insulin resistance, which results in an excessive release of free fatty acid due to the lipolysis. The excessive fatty acid suppresses sugar intake and decomposition in muscle, thereby deteriorating glucose tolerance. Further, the fatty acid is incorporated into the liver and enhances triglyceride synthesis, thereby increasing secretion of triglyceride-rich VLDL cholesterol into the blood. In hyperinsulinemia, excessive production of VLDL occurs. Further, in the state of insulin resistance, lipoprotein lipase activity decreases, which results in decrease in VLDL triglyceride hydrolysis and increase in LDL, LDL cholesterol and triglyceride levels in the blood due to impaired LDL cholesterol catabolism. Further, it is known that decreased synthesis and enhanced catabolism of HDL cholesterol cause the decrease in the amount of HDL cholesterol.

A correlation between insulin resistance and obesity has also been reported. It has been reported that visceral adipose tissue is more strongly associated with the incidence of insulin resistance than subcutaneous adipose tissue. Presumably, free fatty acid released from visceral fat is excessively supplied into the portal vein region, thereby causing insulin resistance in the liver and insulin resistance in the peripheral skeletal muscle as well.

An extract containing an active ingredient according to the present invention actually brought about an increase in the blood HDL cholesterol level, an increase in the blood phospholipid level, a decrease in the blood triglyceride level, an amelioration in the atherogenic index, a decrease in the amount of fat around the kidney, and a suppression of body weight gain (see Examples 1 to 4).

An extract containing an active ingredient according to the present invention actually enhanced the hepatic β oxidation system at the gene level (see Example 5).

An extract containing an active ingredient according to the present invention also exhibited an ameliorating effect on insulin resistance (see Examples 7 and 8).

Accordingly, an active ingredient and isomerized hop extract according to the present invention can be used for treating, preventing or improving diseases or symptoms which can be treated, prevented or ameliorated by activating PPAR.

Examples of the diseases or symptoms which can be treated, prevented or ameliorated by activating PPAR include diabetes (e.g., insulin resistant diabetes, non-insulin dependent diabetes); diabetic complications (for example, ischemic heart diseases such as arteriosclerosis, myocardial infarctions and angina pectoris; cerebral arteriosclerosis such as cerebral infarctions; kidney diseases such as aneurysm and nephrosis; fatty liver or hepatic diseases associated therewith): lipid metabolism abnormalities (e.g., hyperlipidemia, arteriosclerosis, and fatty liver), in particular hyperlipidemia (e.g., hypercholesterolemia, hypo-HDL cholesterolemia, hypertriglyceridemia); insulin resistance and diseases associated therewith (e.g., hyperinsulinemia, impaired glucose tolerance) ; obesity; and body weight gain.

An active ingredient and isomerized hop extract according to the present invention can also be used for ameliorating insulin resistance, improving lipid metabolism, suppressing body weight gain, or slimming (dieting).

The ameliorating effect on insulin resistance is specifically due to a decrease in the insulin concentration, a decrease in the resistin concentration, a decrease in the TNFα concentration, amelioration in glucose tolerance, a decrease in the blood triglyceride and free fatty acid concentrations, miniaturization (normalization) of adipocytes, and the like, which are also included in use of the present invention.

The improving effect on lipid metabolism is specifically due to an increase in the blood HDL cholesterol concentration, amelioration in the atherogenic index, a decrease in the blood triglyceride level, suppression of fat accumulation in the liver, and the like, which are also included in use of the present invention. The improving effect on lipid metabolism brings an antiarteriosclerotic effect, which is also included in use of the present invention.

The suppressing effect on body weight gain is due to suppression of the fat accumulation, in particular suppression of the visceral fat accumulation, which is also included in use of the present invention.

According to the present invention, there is provided use of an active ingredient and isomerized hop extract according to the present invention, for the manufacture of a medicine to be used for treating, preventing or improving diseases or symptoms which can be treated, prevented or ameliorated by activating PPAR.

According to the present invention, there is also provided use of an active ingredient and isomerized hop extract according to the present invention, for the manufacture of a composition to be used for ameliorating insulin resistance, improving lipid metabolism, suppressing body weight gain, or slimming.

Further, according to the present invention, there is provided use of an active ingredient and isomerized hop extract according to the present invention, for the manufacture of a composition for PPAR activation.

According to the present invention, there is provided a method of treating, preventing or improving diseases or symptoms which can be treated, prevented or ameliorated by activating PPAR, comprising administering a therapeutically effective amount of an active ingredient or isomerized hop extract according to the present invention, if necessary, along with pharmaceutically acceptable pharmaceutical additives, to a mammal.

According to the present invention, there is provided a method of ameliorating insulin resistance, improving lipid metabolism, suppressing body weight gain, or slimming, comprising administering a therapeutically effective amount of an active ingredient or isomerized hop extract according to the present invention, if necessary, along with pharmaceutically acceptable pharmaceutical additives, to a mammal.

Further, according to the present invention, there is provided a method of activating PPAR, comprising administering a therapeutically effective amount of an active ingredient or isomerized hop extract according to the present invention, if necessary, along with pharmaceutically acceptable pharmaceutical additives, to a mammal.

### Composition and food

When a composition according to the present invention is provided as a pharmaceutical preparation, it can be produced by mixing an active ingredient or isomerized hop extract according to the present invention with pharmaceutically acceptable additives. A composition according to the present invention can be administered orally or non-orally. Examples of oral formulations include granules, dispersible powders, tablets (including sugar-coated tablets), pills, capsules, syrups, emulsions, and suspensions. Examples of non-oral formulations include injections (e.g., subcutaneous injections, intravenous injections, intramuscular injections, peritoneal injections), intravenous drips, preparations for external use (e.g., nasal formulations, percutaneous agents, ointments), and suppositories (e.g., rectal suppositories, vaginal suppositories). These pharmaceutical preparations can be formulated by a method generally used in this field using pharmaceutically acceptable carriers. Examples of pharmaceutically acceptable carriers include excipients, binding agents, diluents, additives, flavoring agents, buffers, thickening agents, coloring agents, stabilizers, emulsifying agents, dispersing agents, suspending agents, and preservatives; for example, magnesium carbonate, magnesium stearate, talc, sucrose, lactose, pectin, dextrin, starch, gelatin, tragacanth, methylcellulose, sodium carboxymethylcellulose, low-melting wax, and cacao butter can be used as a carrier.

Pharmaceutical preparations can be produced, for example, as follows.

Oral formulations can be produced by adding excipients (e.g., lactose, sucrose, starch, mannitol), disintegrating agents (e.g., calcium carbonate, calcium carboxymethylcellulose), binding agents (e.g., pregelatinized starch, gum arabic, carboxymethylcellulose, polyvinylpyrrolidone, hydroxypropylcellulose), lubricating agents (e.g., talc, magnesium stearate, polyethylene glycol 6000), and the like to an active ingredient, pressing the admixture into an appropriate form, and if necessary, coating for taste masking, enteric film coating or durability using a known method. Examples of coating agents to be used include ethylcellulose, hydroxymethylcellulose, polyoxyethylene glycol, cellulose acetate phthalate, hydroxypropylmethylcellulose phthalate, and Eudragit (methacrylic acid-acrylic acid copolymer; Roehm, Germany).

Formulations for injection can be produced by dissolving, suspending or emulsifying an active ingredient in an aqueous solvent (e.g., distilled water, physiological saline, Ringer's solution) or an oily medium (e.g., vegetable oils such as olive oil, sesame oil, cotton seed oil, and corn oil, or propylene glycol) together with dispersing agents (e.g., Tween 80 (Atlas Powder, USA), HCO 60 (Nikko Chemicals), polyethylene glycol, carboxymethylcellulose, sodium alginate), preservatives (e.g., methylparabene, propylparabene, benzylalcohol, chlorobutanol, phenol), osmosis equilibrating agents (e.g., sodium chloride, glycerin, sorbitol, glucose, invert sugar) and the like. If desired, additives such as solubilizing agents (e.g., sodium salicylate, sodium acetate), stabilizing agents (e.g., human serum albumin) and analgesic agents (e.g., benzalkonium chloride, procaine hydrochloride) may be added.

Pharmaceutical preparations for external use can be produced by formulating an active ingredient into a solid, semi-solid or liquid composition. For example, the abovementioned solid composition can be produced by formulating an active ingredient into a powder as it is or by mixing with addition of excipients (e.g., lactose, mannitol, starch, microcrystal cellulose, sucrose), thickening agents (e.g., natural gums, cellulose derivatives, acrylic acid polymers) and the like to the active ingredient. The abovementioned liquid composition can be produced in almost the same manner as described for injectable preparations. The semi-solid composition is preferably an aqueous or oleaginous gel or ointment. Further, any of these compositions can contain a pH controlling agent (e.g., carbonic acid, phosphoric acid, citric acid, hydrochloric acid, sodium hydroxide), a preservative (e.g., paraoxybenzoic acid esters, chlorobutanol, benzalkonium chloride) and the like. Suppositories can be produced by formulating an active ingredient into an oleaginous or aqueous solid, semi-solid, or liquid composition. Examples of the oleaginous base to be used for such compositions include higher fatty acid glycerides (e.g., cacao oil, Witepsols (Dynamite Nobel)), medium fatty acids (e.g., Miglyols (Dynamite Nobel)), and vegetable oils (e.g., sesame oil, soybean oil, cotton seed oil). Examples of the aqueous base include polyethylene glycols and propylene glycols. Further, examples of the aqueous gel base include natural gums, cellulose derivatives, vinyl polymers, and acrylic acid polymers.

Foods according to the present invention are foods and drinks containing an effective amount of an active ingredient according to the present invention. The expression "containing an effective amount of an active ingredient" herein means that individual foods and drinks contain an active ingredient in the range of the amount described below so that an effective amount of the component can be taken when they are ingested in an ordinary amount. An active ingredient according to the present invention can be blended into foods according to the present invention, as it is or in forms of the abovementioned compositions. More specifically, foods according to the present invention can be those prepared as foods or drinks by using at least one active ingredient or the abovementioned crushed hops or their extract according to the present invention, as they are, those further being admixed with various proteins, sugars, fats, trace elements, vitamins, and the like, those being formulated into a form of liquid, semi-liquid, or solid, or those being added to general foods or drinks.

The term "foods" used in the present invention includes health foods, functional foods, foods for specified health use, and foods for patients.

Further, the form of "foods" is not particularly limited and can be, for example, a drink form.

An active ingredient according to the present invention has effects to ameliorate insulin resistance, improve lipid metabolism, and suppress the accumulation of visceral fat and the weight gain due to fat and cholesterol intake. Accordingly, it is possible to provide foods which simultaneously function in preventing obesity, preventing and ameliorating hyperlipidemia and arteriosclerosis associated with insulin resistance, and preventing diabetic preconditions from developing non-insulin-dependent diabetes, by blending an active ingredient or isomerized hop extract according to the present invention into daily foods, health foods and functional foods taken as supplements, suitably foods containing cholesterol and fat, and the like. Namely, foods according to the present invention can be provided as foods for specified health use, such as foods appropriate for consumers having a relatively high serum cholesterol level and foods suitable for consumers who concern about their blood sugar level.

Examples of such foods and drinks include, but not particularly limited to, those containing carbohydrate such as rice products, noodles, breads, and pastas; various confectionaries including western sweets such as cookies and cakes, Japanese sweets such as buns with a filling and steamed adzuki-bean pastes, candies, chewing gums, and chilled sweets such as yogurt and puddings; various drinks such as juices, soft drinks, and milk drinks; processed foods with eggs; and processed foods (including delicacies) using seafood (squid, octopus, shellfish, eel) or meat (including entails such as liver).

When an active ingredient or isomerized crushed hop or hop extract according to the present invention is used as a food product by admixing with a general food material, it is desirable to prevent the food or drink from being affected by hop bitterness by limiting the amount of use or manipulatively masking.

Since the compositions and foods according to the present invention are hop extract derivatives that have been ingested by humans for many years as foods or drinks, they are low in toxicity and can be used safely for mammals (e.g., humans, mice, rats, rabbits, canines, cats, cattle, horses, pigs, monkeys). The amount of administration or intake for an active ingredient according to the present invention can be determined depending on the recipient, recipient's age, body weight, and symptoms, the time of administration, the type of dosage form, the route of administration, the combination with other medicines, and the like. For example, an active ingredient according to the present invention can be administered as a medicine to an adult orally at a dose ranging from 0.5 to 100 mg/kg body weight (preferably 1 to 50 mg/kg body weight), and non-orally at a dose ranging from 0.05 to 50 mg/kg body weight (preferably 0.5 to 50 mg/kg body weight), in a single dose or in 2 or 3 divided doses daily. Appropriate dosages of medicines having other functions to be used in combination with an active ingredient according to the present invention can be determined based on their individual dosages for clinical use. Further, when taken as foods, an active ingredient according to the present invention can be admixed in the foods so that the amount of its daily ingestion for an adult ranges from 100 to 6000 mg, preferably from 200 to 3000 mg.

### EXAMPLE

The present invention is further illustrated by the following examples that are not intended as a limitation of

### Reference Example

Purification of isohumulone, isoadhumulone, and isocohumulone from a water soluble extract, and purification of humulone and cohumulone from hop extract are shown as reference. Isohumulone, isoadhumulone, and isocohumulone were purified from the water soluble extract described in Example 2 below using HPLC for fractionation (Shimadzu Corp. LC-8 pump, PDA-connected fraction collector system). Conditions used were a mobile phase of 85% methanol-15% 1%-formic acid aqueous solution, a column of YMC-ODS-AQ 25x250 mm, and a flow rate of 20 ml/min. Humulone and cohumulone were purified from the hop extract described in Example 2 using a column of YMC-ODS-AQ 25x250 mm with a mobile phase of 67% methanol-33% 1%-formic acid aqueous solution at a flow rate of 20 ml/min. Fractionated fractions were extracted with ethyl acetate, dried to solid under the reduced pressure, and subjected to weight measurement.

### Example 1

An improving effect on lipid metabolism was evaluated using female C57BL/6 mice. Namely, 5-week-old female C57BL/6NCrj mice (Japan Charles River) (9 or 10 per group) were fed CE2 (Japan Clea) and water ad libitum for 1 week. Then, a high fat, high cholesterol diet (prepared according to the method of Nishina et al., Lipids 28, 599-605, 1993) was administered for 1 week. The composition of the diet is shown in Table 1.

**Table 1:**

| | |
|---|---|
| Unsalted butter | 15% |
| Sucrose | 52.45% |
| Casein | 20% |
| Corn oil | 1% |
| Cellulose | 5% |
| Minerals | 3.5% |
| Vitamins | 1% |
| Choline chloride | 0.25% |
| Cystine | 0.3% |
| Cholesterols | 1% |
| Sodium cholate | 0.5% |

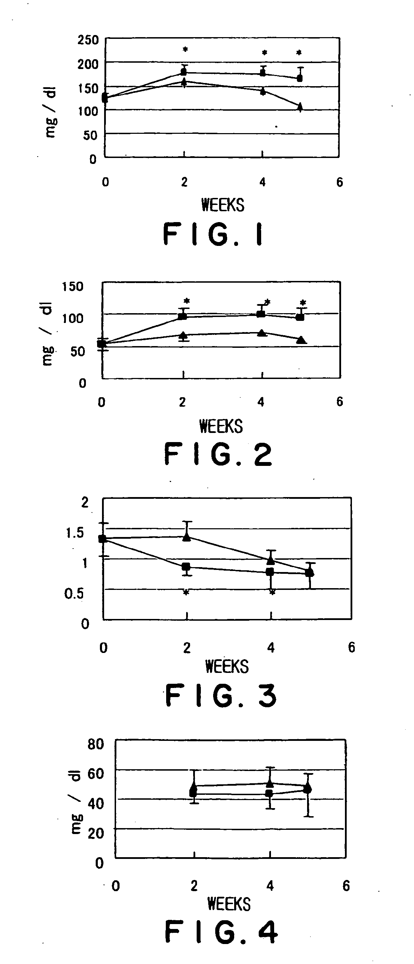

After the preliminary feeding period of 1 week, animals were fasted overnight and then blood samples were collected from each animal via the tail vein using a hematocrit tube. After obtaining plasma, the total cholesterol and the HDL cholesterol were measured using Cholesterol C-II Wako (Wako Pure Chemicals) and HDL-cholesterol-test Wako (Waco Pure Chemicals), respectively, according to the individual manuals attached and then the animals were divided into 2 groups. Mice in one group were fed the abovementioned high fat, high cholesterol diet containing 0.5% (by weight) kettle extract (trade name: Isomerized Kettle Extract (SS. Steiner); an extract prepared from crushed strobili by carbon dioxide gas extraction and isomerization, containing isohumulones as major components as well as lupulones) (except for the first day when the concentration of the extract was 0.2%) ("Kettle" in Figures) ad libitum. Mice in the other control group were fed a mixed diet with addition of 0.5% (by weight) cellulose ("Control" in Figures) ad libitum. The diets were freshly changed every 2 days and the amount of diet intake was recorded. Further, blood samples were collected after overnight fasting on week 2, week 4, and upon dissection. Blood samples were collected from the tail vein on week 2 and week 4, and from the abdominal aorta upon dissection. In addition to the total cholesterol and the HDL cholesterol, the plasma triglyceride level was measured using Triglyceride G Test Wako (Wako Pure Chemicals) according to the attached manual. The atherogenic index was defined as (total cholesterol - HDL cholesterol) /HDL cholesterol. Individual results are shown in Figures 1 to 4.

These results revealed that the HDL cholesterol level was specifically increased 2 weeks and 4 weeks after the start of the administration of Kettle extract, resulting in decreasing the atherogenic index. Further, the Kettle extract tended to decrease the plasma triglyceride level although there was no significant difference (Figure 4).

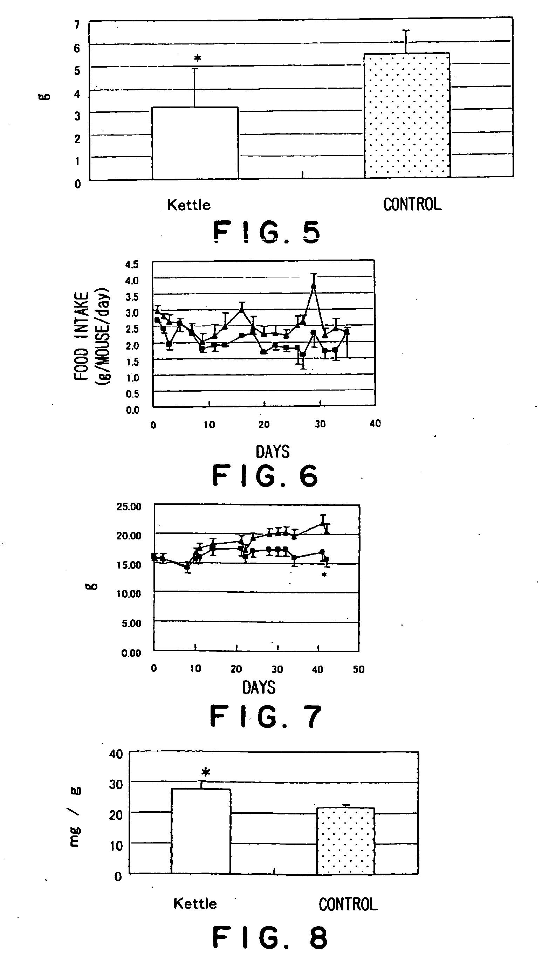

Figure 5 shows the weight of perirenal fat per kg body weight at the time of dissection. The perirenal fat, which is reported to be equivalent to the visceral fat in humans, was revealed to be significantly reduced by the Kettle extract. Further, it was revealed that a marked difference was not observed in the amount of diet intake but a significant difference was observed in the body weight between the two groups, which indicates the effect of the Kettle extract on suppressing body weight gain (Figures 6 and 7) .

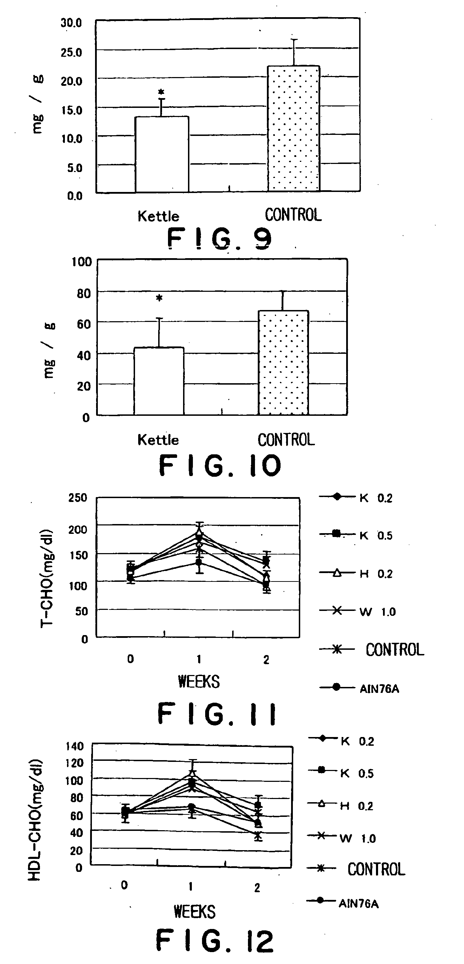

Further, upon dissection, the liver was obtained and the total liver cholesterol, triglyceride, and phospholipid contents were measured. After the dissection, the liver was immediately frozen with liquid nitrogen and then its portion was obtained after crushing and homogenized using a Teflon (trademark) homogenizer with 9-fold by weight of physiological saline under ice cold conditions. Next, lipid was extracted according to the method described in Timothy P. Carr et al., Clinical Biochemistry 26, 39-42, 1993. Namely, 5 ml of chloroform-methanol (2:1) was added to 1 ml of the liver homogenate and the admixture was vigorously stirred, after which 0.5 ml of 0.06 N H₂SO₄ was added and the admixture was stirred again and centrifuged to extract the chloroform phase. A portion of the chloroform phase was dried to solid under nitrogen gas to measure the phospholipid using Phospholipid Test Wako (permanganate ashing method) (Wako Pure Chemicals) (Figure 8). Another portion of the chloroform phase was mixed with chloroform containing 1% Triton-X100, after which the mixture was dried to solid under nitrogen gas and the solid was suspended in water to measure the total cholesterol and triglyceride by the abovementioned method. The results are shown in Figures 9 and 10, respectively. The results showed that the Kettle extract significantly decreased the liver cholesterol and triglyceride contents and significantly increased the phospholipid content.

Further, liver dysfunction index enzymes, GOT (glutamic oxaloacetic transaminase), GPT (glutamic pyruvic transaminase), and ALP (alkaline phosphatase), were measured using a Hitachi 7170 automatic plasma analyzer (Hitachi, Ltd.) according to the attached manual, which confirmed that all figures were decreased in the group administered with the Kettle extract, showing no incidence of liver dysfunction.

From the results above, it was revealed that in animal models fed a high fat and high cholesterol diet, an isomerized hop extract mainly consisting of isohumulones and lupulones is highly effective in improving lipid metabolism by increasing blood HDL cholesterol, decreasing the atherogenic index, and suppressing accumulation of triglyceride and cholesterol in the liver, in suppressing fat accumulation, and in suppressing body weight gain caused by the intake of high fat and high cholesterol diet.

### Example 2

C57BL/6 mice were fed a high fat and high cholesterol diet in Example 1 and used for 2 week evaluation of the effect of change in the amount using a mixed diet with 0.2% or 0.5% Kettle extract (described in Example 1) and the effect of a hop extract (trade name: CO2 Pure Resin Extract (Hopsteiner), an extract of humulones and lupulones from hop strobili) and a water soluble extract (trade name: ISOHOPCO2N (English Hop Products) obtained by extracting humulones from hop strobili, isomerizing the humulones into isohumulones and then transforming them into potassium salts, containing almost no humulones or lupulones). Further, mice in control groups were fed a normal diet AIN76A (Dyets) ad libitum. Namely, 5-week-old C57BL/6NCrj female mice (8 or 9 per group) were fed CE2 and water for 1 week ad libitum. Then, they were administered with a high fat and high cholesterol diet (prepared according to the method described in Example 1) for 1 week. Diets used in this Example were those solidified into pellets by adding water and stored in a freezer. Further, the diets were freshly changed and the amount of diet intake was recorded everyday. After the preliminary feeding period of 1 week, mice were fasted overnight to take blood samples from the tail vein using a hematocrit tube, the total cholesterol and the HDL cholesterol were quantitatively measured according to the method of Example 1, and the animals were so divided into groups as to minimize the variation between the groups. Next, the mice were fed with diets containing 0.2% and 0.5% by weight Kettle extract (K 0.2, K 0.5), 0.2% hop extract (H 0.2), and 1% water soluble extract (W 1.0), respectively, which were prepared by mixing the extracts in the specified concentrations with a high fat and high cholesterol diet ad libitum. Blood samples were collected one week after from the tail vein under non-fasting conditions and two weeks after from the abdominal vein under fasting conditions.

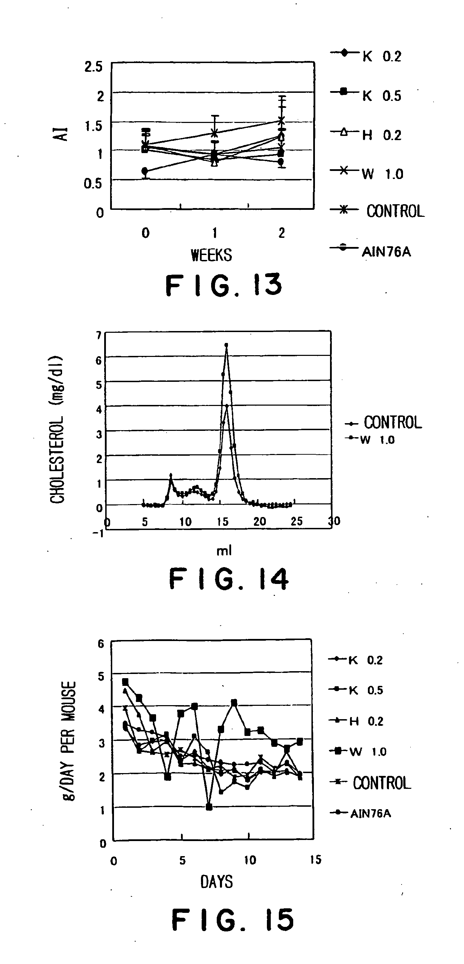

Results for the total cholesterol, HDL cholesterol, and the atherogenic index are shown in Figures 11, 12 and 13, respectively. It was revealed that the Kettle extract (K 0.2, K 0.5) dose-dependently increased the HDL level and ameliorated the atherogenic index. Further, it was revealed that the non-isomerized hop extract also improve lipid metabolism. Further, plasma samples (150 µl) from mice fed the control diet and the water soluble extract were subjected to gel filtration chromatography. The result is shown in Figure 14. The method of Y.C. Ha et al. (Journal of Chromatography 341, 154-159, 1985) was used. Namely, the chromatography was carried out using a Superose 6B column (Amersham-Pharmacia) and a P-500 pump (Amersham-Pharmacia) with a mobile phase of 0.15 M NaCl, 0.01% EDTA-Na₂, and 0.02% NaN₃, pH 7.2, at a flow rate of 0.33ml/min. Fractions of 5 ml were collected. The total cholesterol was measured for each fraction.

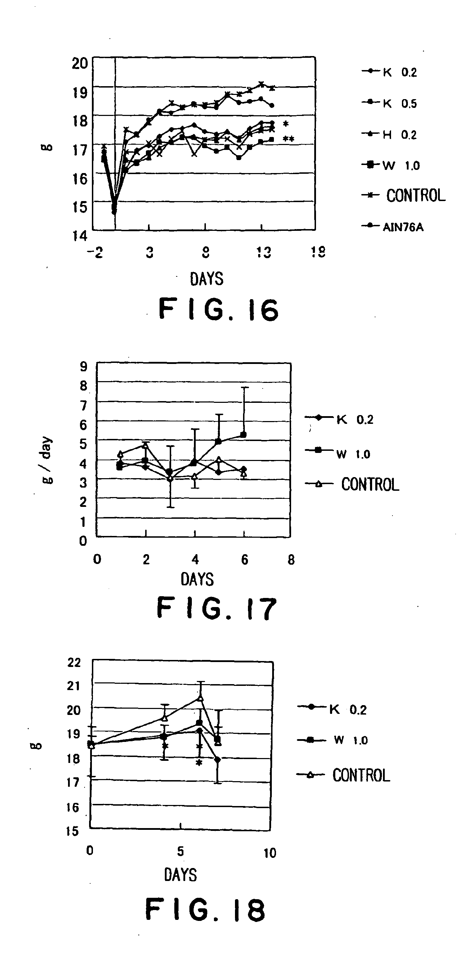

As a result, it was revealed also from this method that the water soluble extract specifically increased only the HDL fraction eluted after an elution volume of 15 ml. Further, daily changes in the amount of diet intake and body weight per mouse are shown in Figure 15 and Figure 16, respectively. Despite the fact that there was no difference in the amount of diet intake between groups, except for the group with the water soluble extract (W 1.0), the weight gain was significantly decreased in the groups administered with the Kettle extract (K 0.2, K 0.5), the water soluble extract (W 1.0) and the hop extract (H 0.2) as compared to that in the groups with the AIN76A and the control diet.

From the results above, it was revealed that in addition to the isomerized hop extract mainly consisting of isohumulones and lupulones, both the water soluble extract mainly consisting of isohumulones and the hop extract mainly consisting of humulones and lupulones were effective in improving lipid metabolism, for example, by increasing the blood HDL cholesterol level and decreasing the atherogenic index, and in suppressing body weight gain.

### Example 3

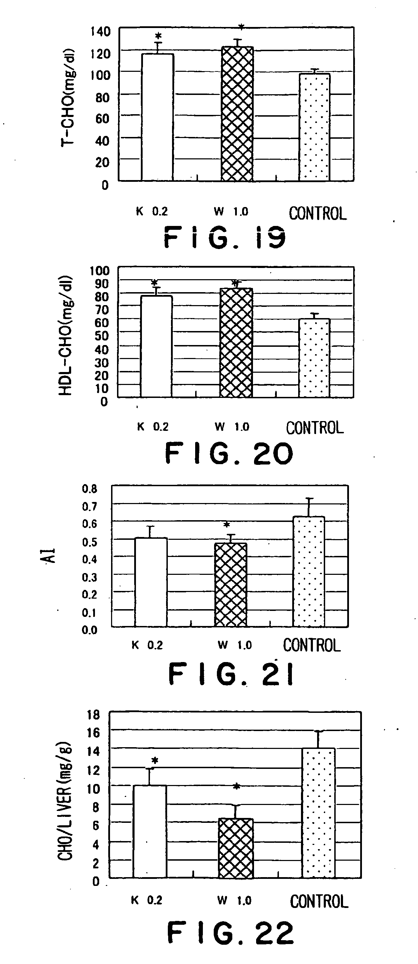

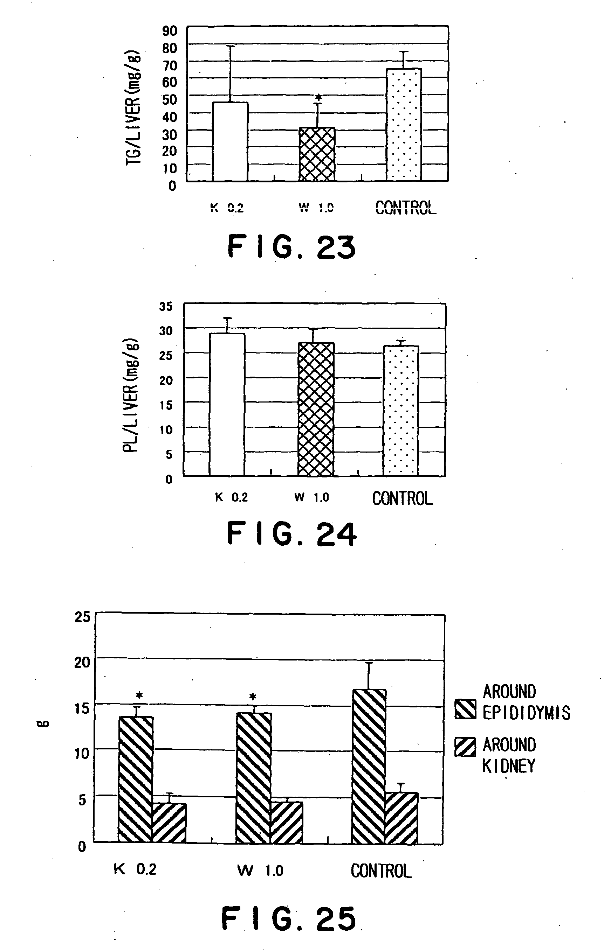

An improving effect on lipid metabolism was evaluated using C57BL/6 male mice. Namely, 5-week-old C57BL/6NCrj male mice (purchased from Japan Charles River) (5 or 6 per group) were fed CE2 (Japan Clea) and water for 1 week ad libitum. Then, a diet was first prepared by adding 0.2% cholesterol to AIN76A (Dyets) and the following supplements were added to this diet to prepare experimental diets according to the method described in Example 2: 1% water soluble extract (Example 2) ("W 1.0" in Figures) for one group, 0.2% Kettle extract (Example 1) ("K 0.2" in Figures) for another group, and 0.5% cellulose for a control group. Each diet was fed to the animals ad libitum. The amount of daily intake per animal and change in body weight are shown in Figures 17 and 18, respectively. It was revealed that in the group fed the water soluble extract (W 1.0), the weight gain was significantly reduced the day before dissection as compared to the control group, although the amount of diet intake tended to increase. After one week, the whole blood was taken from the abdominal vein after overnight fasting, and the total cholesterol and the HDL cholesterol were measured as described in Example 1. The result revealed that the water soluble extract (W 1.0) specifically increased the HDL cholesterol level and significantly decreased the atherogenic index (Figures, 19, 20 and 21). Further, the amount of cholesterol, triglyceride, and phospholipid per g liver was measured, which showed that the water soluble extract (W 1.0) significantly reduced the amounts of cholesterol and triglyceride, and the Kettle extract (K 0.2) reduced them (Figures 22, 23 and 24). Further, it was shown that the amount of perirenal fat (by weight) per kg body weight was significantly decreased and the amount of fat (by weight) around the epididymis tended to be decreased at the time of dissection by the water soluble extract (W 1.0) and the Kettle extract (K 0.2) (Figure 25).

From the results above, it was revealed that also in animal models fed a diet with addition of cholesterol at a low concentration, the isomerized hop extract mainly consisting of isohumulones and lupulones and the water soluble hop extract mainly consisting of isohumulones are highly effective in improving lipid metabolism by increasing the HDL cholesterol level in the blood, decreasing the atherogenic index, and suppressing the accumulation of triglyceride and cholesterol in the liver, in suppressing the accumulation of visceral fat, and in suppressing body weight gain.

### Example 4

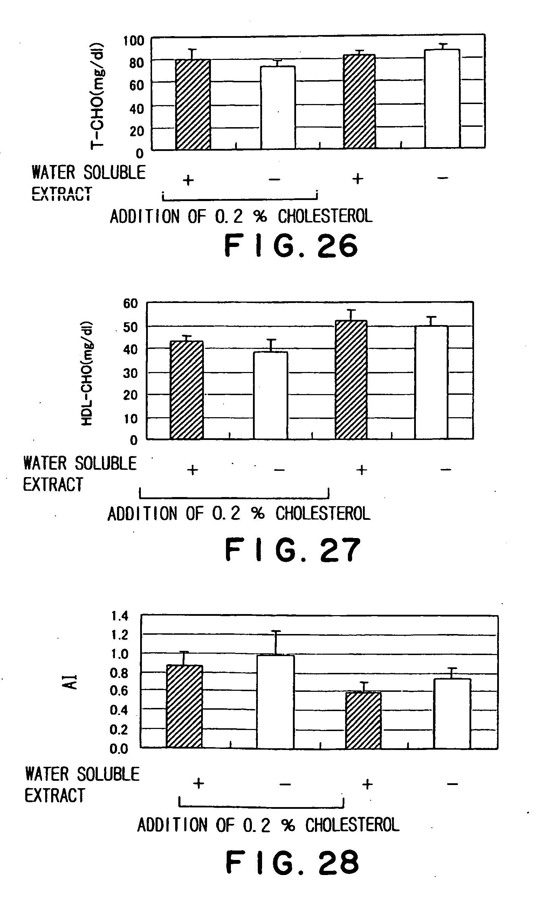

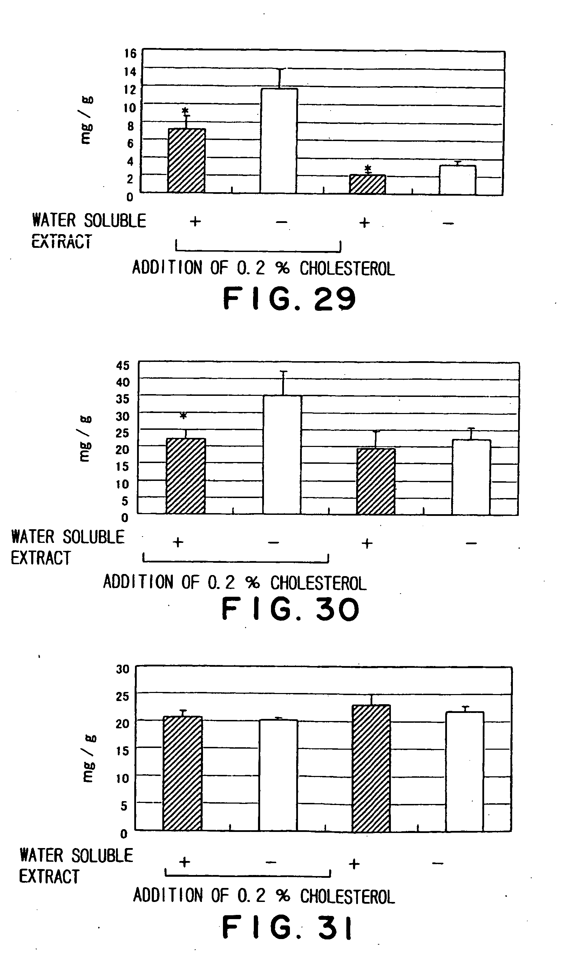

An improving effect on lipid metabolism was evaluated using C57BL/6 female mice. Namely, 5-week-old C57BL/6NCrj female mice (Japan Charles River) (5 to 6 per group) were fed CE2 (Japan Clea) and water for 1 week ad libitum. Then, animals were divided into four groups: a group fed AIN76A (described in Example 2) supplemented with 0.2% cholesterol and 0.3% cellulose, a group fed AIN76A supplemented with 0.2% cholesterol and 1% water soluble extract, a group fed AIN76A supplemented with 0.3% cellulose, and a group fed AIN76A supplemented with 1% water soluble extract. The diets were prepared and administered as described in Example 2. After one week, animals were dissected under non-fasting conditions, the whole blood was collected from the abdominal vein, and the total cholesterol and the HDL cholesterol were measured as described in Example 1. It was confirmed that the water soluble extract increased the HDL cholesterol level associated with a decrease in atherogenic index, although the differences were not significant (Figures 26, 27, and 28). Further, cholesterol, triglyceride, and phospholipid contents per g liver were measured, which confirmed that the water soluble extract significantly decreased the cholesterol content under the conditions with no cholesterol added and significantly decreased the cholesterol and triglyceride contents under the conditions with cholesterol added (Figures 29, 30, and 31).

From the results above, it was revealed that also in animal models fed a diet without cholesterol added, the water soluble extract mainly consisting of isohumulones was effective in improving lipid metabolism, for example, by increasing the HDL cholesterol level in the blood, decreasing the atherogenic index, and suppressing accumulation of triglyceride and cholesterol in the liver.

### Example 5

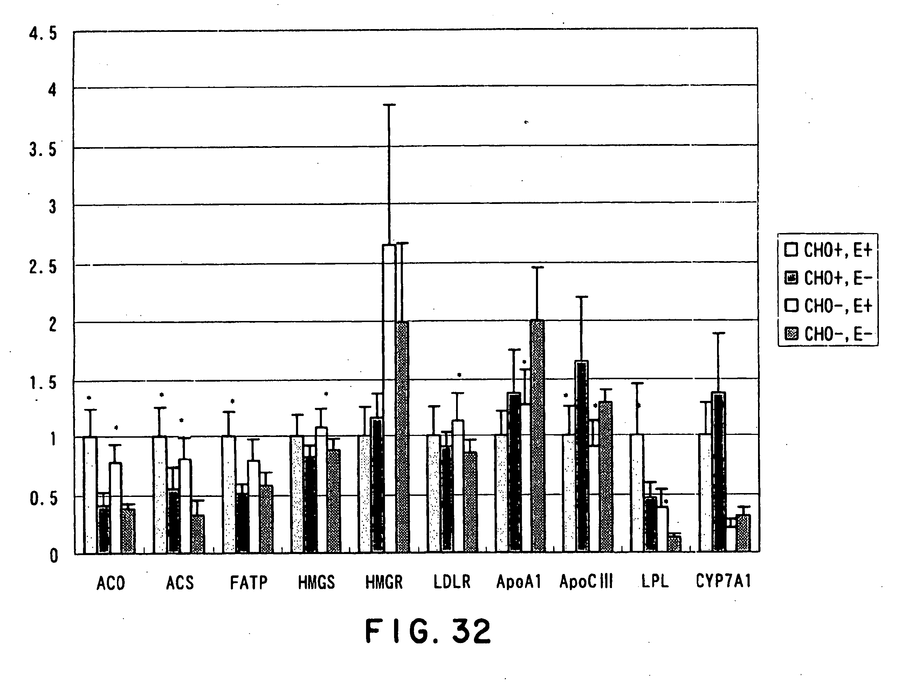

In Example 4, the liver was frozen for storage immediately after dissection using liquid nitrogen. RNA was obtained from about 100 mg of liver tissue using Isogen (Nippon Gene) according to the attached manual. The amount of RNA was measured using a spectrophotometer, after which annealing with oligo dT was carried out using a Thermo Script TM RT-PCR system (Lifetech Oriental) according to the attached manual, the RNA was reverse transcribed, and thus the cDNA was obtained. The resulting cDNA was analyzed for acyl-CoA oxidase (ACO) using
5'-ATCTATGACCAGGTTCAGTCGGGG-3' (SEQ ID NO: 1) as a sense primer and
5-CCACGCCACTTCCTTGCTCTTC-3' (SEQ ID NO: 2) as an antisense primer;
for acyl-CoA synthetase (ACS) using
5'-GGAACTACAGGCAACCCCAAAG-3' (SEQ ID NO: 3) as a sense primer and
5'-CTTGAGGTCGTCCATAAGCAGC-3' (SEQ ID NO: 4) as an antisense primer;
for fatty acid transport protein (FATP) using
5'-TGCTAGTGATGGACGAGCTGG-3' (SEQ ID NO: 5) as a sense primer and
5'-TCCTGGTACATTGAGTTAGGGTCC-3' (SEQ ID NO: 6) as an antisense primer;
for 3-hydroxy-3-methylglutaryl coenzyme A synthase (HMGCS) using 5'-CCTTCAGGGGTCTAAAGCTGGAAG-3'(SEQ ID NO: 7) as a sense primer and
5'-CAGCCAATTCTTGGGCAGAGTG-3'(SEQ ID NO: 8) as an antisense primer;
for 3-hydroxy-3-methylglutaryl coenzyme A reductase (HMGCR) using
5' -TTGGCCTCCATTGAGATCCG-3' (SEQ ID NO: 9) as a sense primer and
5'-GATCTTGTTGTTGCCGGTGAAC-3' (SEQ ID NO: 10) as an antisense primer;
for low density lipoprotein receptor (LDLR) using
5'-CATCAAGGAGTGCAAGACCAACG-3' (SEQ ID NO: 11) as a sense primer and
5'-CACTTGTAGCTGCCTTCCAGGTTC-3' (SEQ ID NO: 12) as an antisense primer;
for apo-AI using
5'-TGTATGTGGATGCGGfC;AAAGAC-3' (SEQ ID NO: 13) as a sense primer and
5'-TCATCTCCTGTCTCACCCAATCTG-3' (SEQ ID NO: 14) as an antisense primer;
for apo-CIII using
5' -AGGGCTACATGGAACAAGCCTC-3' (SEQ ID NO: 15) as a sense primer and
5'-CGACTCAATAGCTGGAGTTGGTTG-3' (SEQ ID NO: 16) as an antisense primer;
for lipoprotein lipase (LPL) using
5'-GTTTGGCTCCAGAGTTTGACCG-3' (SEQ ID NO: 17) as a sense primer and
5' -CATACATTCCCGTTACCGTCCATC-3' (SEQ ID NO: 18) as an antisense primer; and
for cholesterol alpha-7-hydroxylase (CYP7A1) using 5'-ACGGGTTGATTCCATACCTGGG-3' (SEQ ID NO: 19) as a sense primer and
5'-TGTGTCCAAATGCCTTCGCAG-3' (SEQ ID NO: 20) as an antisense primer.
As an internal standard gene, the acidic ribosomal phosphoprotein PO (acidic ribosomal protein 36B4) gene was used. For 36B4,
5'-CCAAGCAGATGCAGCAGATCC-3' (SEQ ID NO: 21) was used as a sense primer and 5'-CAGCAGCTGGCACCTTATTGG-3' (SEQ ID NO: 22) was used as an antisense primer. Each mRNA was quantitatively measured based on cDNA using a Light Cycler (Roche) and FastStart DNA Master SYBR Green I (Roche) as a reaction kit, according to the attached manual. The result of the expression of each gene is shown in Figure 32, in which the amount of the expression of the internal standard under conditions with addition of the water soluble extract and with cholesterol added is set as 1. The results of two-way analysis of variance confirmed correlations of the water soluble extract with ACO, ACS, FATP, Apo-AI, Apo-CIII, and LPL. These genes were reported to show the similar expression behavior by administration of fenofibrate, a PPARα ligand (The Molecular Atherosclerology, Nobuhiro Morisaki, et al., Medical Review; J. Clin. Invest. 1996, 97:2408-2416, Laurence Berthou et al.).

The results above suggested that the improving effect on lipid metabolism due to isohumulone intake was caused by acceleration of hepatic β-oxidation system; this change in the gene expression might probably be caused due to the PPAR-α agonist action by isohumulones.

### Example 6

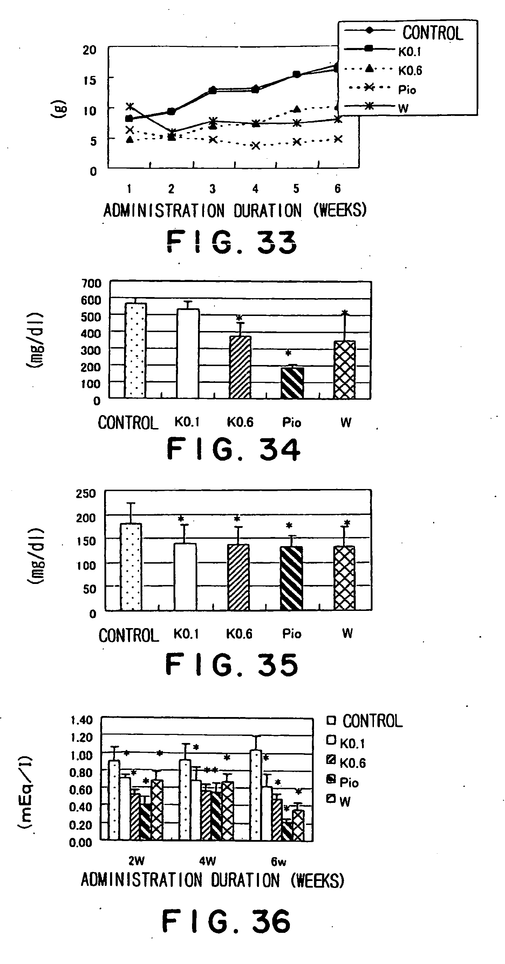

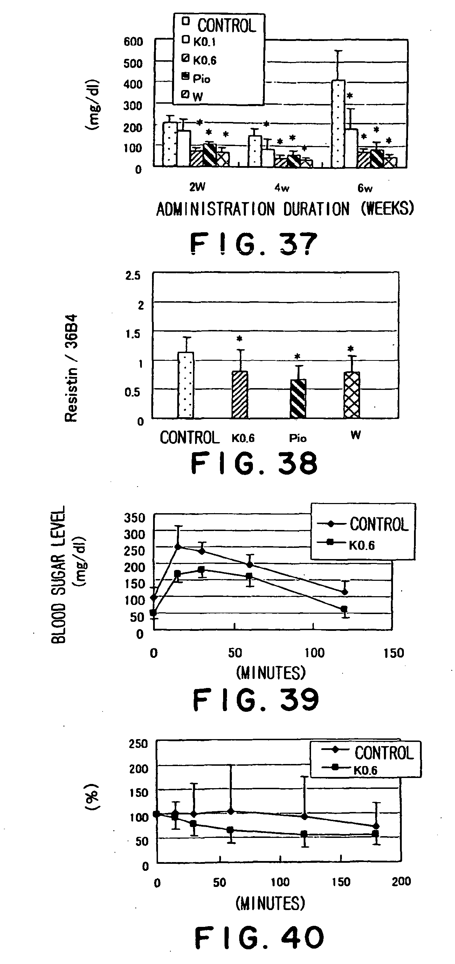

An ameliorative effect on insulin resistance was evaluated using KKA^{y} mice (males). Namely, 5-week-old KKA^{y}/Ta Jc1 mice (Japan Clea) (8 or 9 per group) were fed CE-2 (Japan Clea) and water for 1 week for habituation ad libitum. Then, the diet was replaced by a powdered diet based on the AIN93 (standard composition according to US National Institute of Nutrition) which was prepared using purified materials. The experimental animals were divided into a control group (AIN93 diet only); two groups fed with addition of 0.1% and 0.6% by weight Kettle extract (group K 0.1 and K 0.6); a group fed with addition of 0.05% (by weight) powered pioglitazone (trade name: Actos, Takeda) (group Pio) and a group fed with addition of 1.2% (by weight) water soluble extract (group W). The diet mixed with the water soluble extract was prepared by adding an aqueous extract dilution to the powdered diet for formulation. Animals in the control group, group K 0.1, group K 0.6 and group Pio were fed 5 g of the powdered diet every day. This is the amount that each animal can eat a day; in this way of feeding, the amount of diet intake can be consistent between the groups. Animals in group W were fed ad libitum. The amount of water intake was measured weekly. On week 2 and week 4 after the start of experimental feeding, animals were fasted for about 15 hours and then blood samples were collected from the tail vein to measure the triglyceride and free fatty acid levels in the blood. The triglyceride concentration was measured using a Lipidos Liquid (Toyobo) and the free fatty acid concentration was measured using an NEFA C-Test Wako (Wako Pure Chemicals). On week 4 when blood samples were collected, the fasting blood sugar level was also measured. On week 5, animals in the control group, group K 0.1, group K 0.6, group Pio, and group W were fed 10 g of diet once and blood samples were collected from the tail vein on the following day to measure the non-fasting blood sugar level. The blood sugar level was measured using a Glutest Sensor (Sanwa Kagaku Kenkyusho Co., Ltd.). On week 6, animals in the control group, group K 0.1, group K 0.6, group Pio, and group W were fed 10 g of diet and dissected under non-fasting conditions to collect fat tissue around the epididymis and whole blood from the abdominal vena cava. The levels of triglyceride and free fatty acid in the blood were also measured at the time of dissection. The total RNA was prepared from fat around the epididymis using ISOGEN (Nippon Gene). The amount of expression of the resistin gene was measured by the quantitative RT-PCR method using the total RNA thus prepared. Reverse transcription reaction was carried out using a thermoscript RT-PCR system (Gibco BRL), and the quantitative PCR was carried out using a LightCycler (Roche) and a LightCycler-FastStart DNA Master SYBR Green I (Roche). The sequences of primers used were
5' -CGTGGGACATTCGTGAAGAAAAAG-3' (SEQ ID NO: 23) and
5'- TGTGCTTGTGTGTGGATTCGC-3' (SEQ ID NO: 24).
The amount of resistin expression was standardized by the measurement using primers of acidic ribosomal protein 36B4:
5'-CCAAGCAGATGCAGCAGATCC-3'(SEQ ID NO: 25) and
5'- CAGCAGCTGGCACCTTATTGG-3'(SEQ ID NO: 26).
The amount of water intake per day during rearing is shown in Figure 33. It is known that the water intake increases in KKA^{y} mice exhibiting hyperglycemia caused by insulin resistance and decreases in mice with ameliorated resistance (Kakuda et al., Biosci. Biotech. Biochem., 60(2), 204-208, 1996); a decrease in water intake was confirmed in group K 0.6 and group W similarly to that in group Pio, the group fed a diabetes ameliorating agent. The non-fasting blood sugar level on week 5 and the fasting blood sugar level on week 4 are shown in Figures 34 and 35, respectively. Both non-fasting and fasting blood sugar levels in group K 0.6 and group W were significantly reduced similarly to that in group Pio, as compared to that in the control group. The levels of free fatty acid and triglyceride in the blood on week 2 and week 4 under fasting conditions and on week 6 (upon dissection) under non-fasting conditions are shown in Figures 36 and 37, respectively. The blood lipid levels in groups K 0.1, K 0.6 and W were significantly decreased as compared to that in the control group. As shown in Figure 38, the amount of resistin gene expression was significantly decreased in group K 0.6 and group W as compared to that in the control group.

The results shown in Figures 33 to 38 above all indicated that insulin resistance of KKA^{y} mice was reduced by the isomerized hop extract mainly consisting of isohumulones and lupulones and the water-soluble hop extract mainly consisting of isohumulones, which revealed that these extracts are markedly effective in ameliorating insulin resistance.

### Example 7

After 5-week-old KKA^{y} mice were reared for 1 week for habituation (as described in Example 6), they were divided into a control group fed the standard diet (described in Example 6) and group K 0.6 fed with addition of 0.6% Kettle extract (as described in Example 6), 6 mice per group, and were fed the diets and water for 12 weeks ad libitum. On week 12, the animals were fasted for 5 hours and then subjected to a glucose tolerance test as follows. Namely, the blood sugar level at time zero was measured as described in Example 6 before the administration of glucose. Then, a 20% (w/v) glucose aqueous solution was administered to each animal using an oral sonde so as to make the amount of glucose administered to be 2 g per kg body weight, and the blood sugar level was measured 15, 30, 60, and 120 minutes after the administration. An insulin sensitivity test was carried out 1 week after the glucose tolerance test as follows. Namely, the blood sugar level of each mouse was measured at time zero before insulin administration under non-fasting conditions after the collected blood sample was diluted 2 times with physiological saline, in the same manner as the glucose tolerance test. Then, a pig insulin solution prepared with physiological saline at 75 mU/ml was administered intraperitoneally so as to make the amount of insulin administered to be 0.75 U per kg body weight. The blood sugar level was measured 15, 30, 60, 120, and 180 minutes after the administration.

As shown in Figure 39, it was revealed that the glucose tolerance was ameliorated in group K 0.6 as compared to that in the control group. Further, as shown in Figure 40, insulin resistance tended to be ameliorated in group K 0.6 as compared to that in the control group which exhibited severe resistance. The results above revealed that the isomerized hop extract mainly consisting of isohumulones and lupulones has insulin resistance ameliorating activity.

### Example 8

It is known that C57BL/6 mice fed high fat diet exhibit obesity and hyperglycemia (Ikemoto et al., Metabolism 45(12), 1539-46, 1996). Accordingly, the action of hop extracts on the incidence of diet-derived insulin resistance was studied. Namely, 5-week-old C57BL/6 NCrj mice (Japan Charles River) (8 per group) were fed CE-2 (Japan Clea) and water for 1 week for habituation ad libitum. The diet was then replaced by a high fat diet (Table 2) prepared using purified materials.

**Table 2: Composition of high fat diet (figures are % by weight)**

| | |
|---|---|
| Safflower oil | 33.5 |
| Casein | 29.0 |
| Sucrose | 23.3 |
| Vitamin mix (Oriental Yeast) | 1.45 |
| Mineral mix (Oriental Yeast) | 5.08 |
| Cellulose powder | 7.25 |
| L-Cystine | 0.44 |

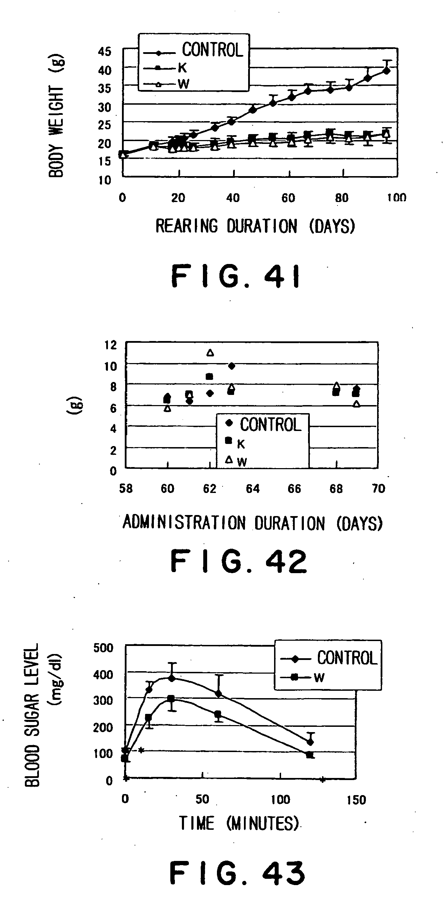

The experimental animals were divided into a group fed the high fat diet, a group fed the diet with addition of 0.3% by weight Kettle extract(group K), and a group fed the diet with addition of 0.6% by weight water soluble extract(group W). The diets and water were fed during the feeding period ad libitum and the diets were freshly changed everyday. The body weight was measured everyday after the start of the rearing. As shown in Figure 41, the body weight gain was more moderate in group K and group W than in the control group. Further, the amount of daily diet intake was measured on day 60 after the start of rearing and thereafter (6 times), which indicated no significant difference in the amount of diet intake between the groups as shown in figure 42. The results above confirmed that the hop extracts have activity in suppressing obesity caused by high fat diet feeding. Further, on day 84 after the start of rearing, 4 animals each from the control group and group W were subjected to a glucose tolerance test. The test was carried out according to the method described in Example 7. The results of glucose tolerance test showed that both the maximum blood sugar level and 120-minutes blood sugar level in group W were lower than those in the control group, as shown in Figure 43.

The results above confirmed that the water soluble hop extract mainly consisting of isohumulones have activity further to ameliorate impaired glucose tolerance.

### Example 9

Construction of a PPARγ agonist screening system and results of activity evaluation are shown below.

In order to construct a human PPARγ expression plasmid, a PPARγ ORF was cloned from the human heart cDNA library (Gibco). After the sequence was confirmed, the cloned ORF was ligated to the NheI-SalI site of the expression vector pCI neo (Promega). The sequences of primers used for the cloning were as follows:
5' GCTAGCATGGTGGACACGGAAAGCCC 3'(SEQ ID NO: 27) and
5' GTCGACAGTACATGTCCCTGTAGATCTC 3' (SEQ ID NO: 28).

Next, in order to construct a reporter plasmid, oligo DNAs having 3 copies of PPRE was constructed and inserted at the KpnI-BglII site of the firefly luciferase reporter vector pGL3-promoter vector (Promega), after which the sequences were confirmed. The sequences of oligo-DNAs containing PPRE are as follows:
5'CAGGGGACCAGGACAAAGGTCACGTTCGGGAAGGGGACCAGGACAAAGGTCACGT 3' (SEQ ID NO: 29) and
5'GATCTTCCCGAACGTGACCTTTGTCCTGGTCCCCTTCCCGAACGTGACCTTTGTC 3' (SEQ ID NO: 30).

CV-1 cells were transfected with the abovementioned plasmid along with the renilla luciferase reporter vector pRL-SV40 vector for compensation (Promega) using Lipofect AMINE (Gibco). The CV-1 cells used were cultured at a concentration of 5 × 10⁴ cells/ml in 2 ml of DMEM (Gibco) supplemented with 10% fetal calf serum (Gibco) and penicillin-streptomycin (10000 units and 1 mg/ml, respectively; Gibco) on a 12-well plate at 37°C in an atmosphere of 5% CO₂ on the day before transfection. After the transfection, cells for the positive control were cultured in the abovementioned DMEM medium containing 1 µM pioglitazone (Takeda). After cultivating for 48 hours, the cells were recovered and the lysate was prepared using a Dual-Luciferase reporter assay system (Promega) to measure firefly luciferase and renilla luciferase activity using a luminometer (Luminous CT-9000D, DIA-IATRON). Relative luciferase activity was obtained by dividing the value for firefly luciferase activity by the value for renilla luciferase activity.

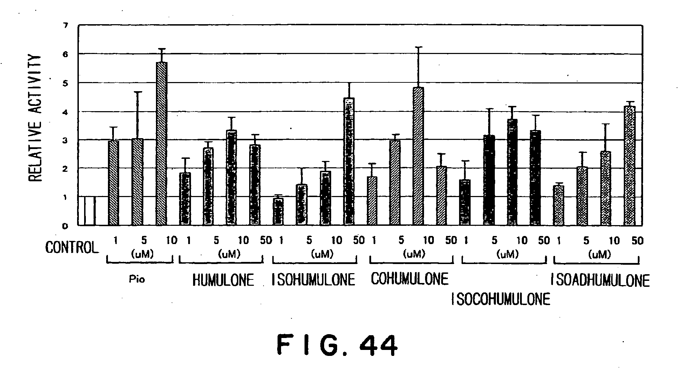

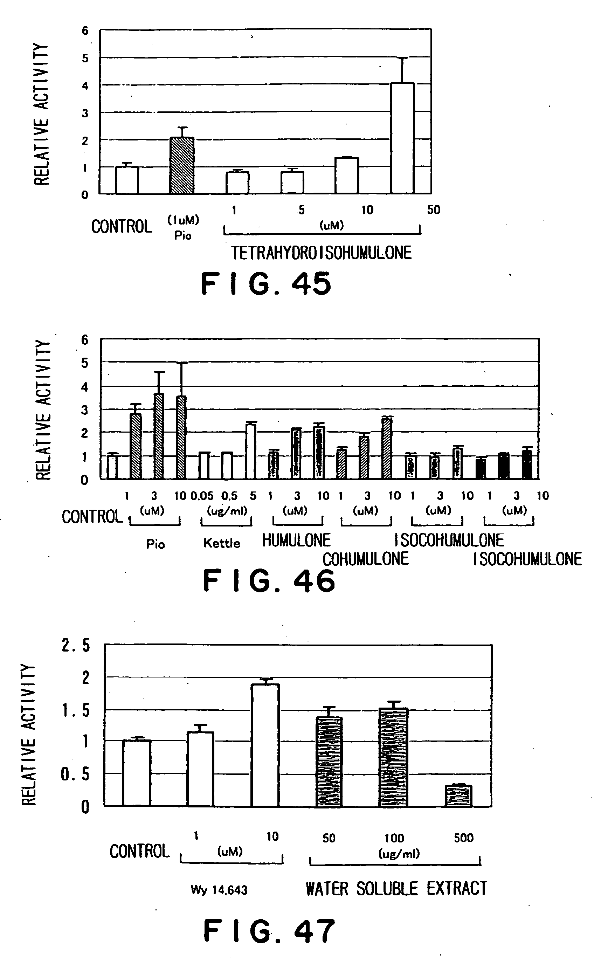

Using the assay system described above, a series of humulone compounds (humulones, cohumulones, isohumulones, isocohumulones, and isoadhumulones) were tested for their PPARγ/RXR alpha activating activity. When tested at concentrations of 1, 5, 10, and 50 µM, all humulone compounds exhibited the activity, and the activity was almost equivalent to that with 1 µM pioglitazone at a concentration of 10 µM (Figure 44). Further, similar activity was observed with tetrahydroisohumulone (Figure 45).

### Example 10

Construction of a PPARγ agonist screening system consisting of a fused protein and results of activity evaluation are shown.

In order to construct a human PPARγ expression plasmid, a PPARγ ligand binding domain (LBD; 204a.a.-505a.a) was cloned from the human heart cDNA library (Gibco). After the sequence was confirmed, the cloned ORF was ligated to the BamHI-Kpn1 site of the expression vector pBind (Promega) to construct the expression vector pGR-Ga14-PPARγ which expresses a fused protein of PPARγ with yeast Ga14 protein. The sequences of primers used for the cloning are as follows:
5' GGATCCTTTCTCATAATGCCATCAGGTTTG 3' (SEQ ID NO: 31) and
5' GGTACCTTCCGTGACAATCTGTCTGAG 3' (SEQ ID NO: 32).

Next, an N terminal sequence (1a.a-76a.a) of the human glucocorticoid receptor (GR) was ligated to the N-terminal of the Gal4 region of pBind so that the reading frame coincided with Gal4. The GR was cloned from the heart cDNA library (Gibco) using PCR. The sequences of primers used for the cloning are as follows:
5' GCTAGCATGGACTCCAAAGAATCATTAAC 3' (SEQ ID NO: 33) and
5' TGGCTGCTGCGCATTGCTTA 3' (SEQ ID NO: 34).

As a reporter plasmid, firefly luciferase expression vector pG51uc (Promega) having 5 copies of the Gal4 binding site introduced into the promoter region was used. CV-1 cells were transfected with the pGR-Gal4-PPARγ and the pG5luc using Lipofect AMINE (Gibco). After the transfection, the medium was replaced with a medium (DMEM, Gibco) with addition of a test sample or control (pioglitazone), and cells were recovered after cultivation for 48 hours. After the cell recovery, cell lysate was prepared using a Dual-Luciferase reporter assay system (Promega) to measure firefly luciferase activity using a luminometer (Luminous CT-9000D, DIA-IATRON). Further, the protein concentration of the cell lysate was measured using a Dc Protein assay (BIO-RAD) to standardize the value of firefly luciferase activity for the protein concentration. The result was expressed as a relative value by setting the value of the negative control to be 1.

Using the assay system described above, the Kettle extract and a series of humulone compounds (humulones, cohumulones, isohumulones, and isocohumulones) were studied for their PPARγ activating activity. When studied using the Kettle extract at a concentration of 0.05, 0.5, and 5 µg/ml and the humulone compounds at a concentration of 1, 3, and 10 µM, the activity was confirmed with all the samples tested similarly to Example 9 (Figure 46).

### Example 11

Construction of a PPARα agonist screening system and results of activity evaluation are shown.

Also for PPARγ, a screening system was constructed in the same manner as for PPARγ, except for the conditions described below. The sequences of primers used for the cloning are as follows:
5' GGATCCTTTCACACAACGCGATTCGTTTTG 3' (SEQ ID NO: 35) and
5' GGTACCGTACATGTCCCTGTAGATCTC 3' (SEQ ID NO: 36).

Transfection was carried out also as described for the PPARγ system, except that Wy 14,643 (Wako Pure Chemicals) was used as a control for PPARα.

Using the abovementioned assay system, the water soluble extract was studied at concentrations of 50, 100, and 500 µg/ml, which confirmed that the water soluble extract had an ability to activate PPARα at concentrations of 50 and 100 µg/ml (Figure 47).

### Example 12: Example of blending into food

Glutinous starch syrup (300 g) was melted into 650 g of sugar by heating at 150°C and then cooled to 120°C, after which 10 g of citric acid was added, then 30 g of the water soluble extract described in Example 2 and 10 g of essence were added, the resulting admixture was stirred, homogenized, formed, and cooled to produce candies.

### Example 13

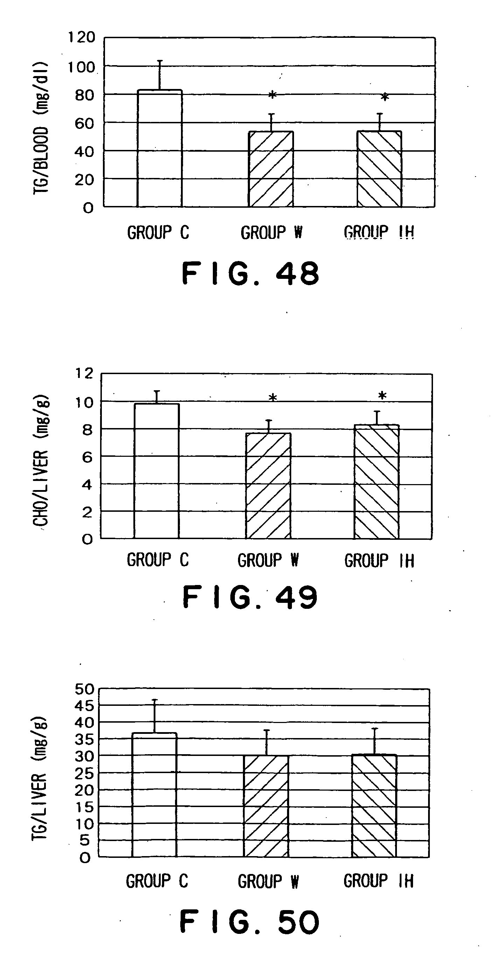

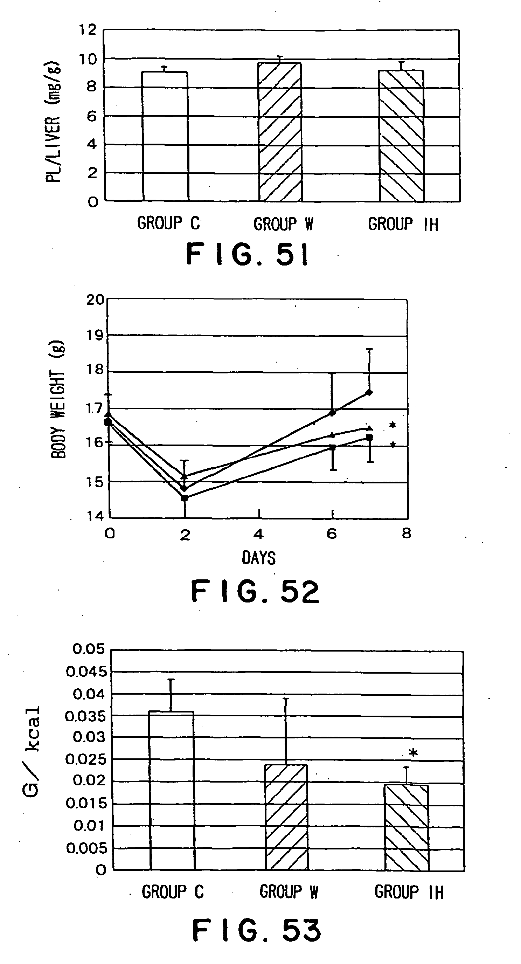

Lipid metabolism-improving effect of a fraction containing fractionated cis-isohumulone, trans-isohumulone, cis-isoadhumulone, trans-isoadhumulone, cis-isocohumulone, and trans-isocohumulone was evaluated using C57BL/6 mice (females). Namely, from the water soluble extract (described in Example 2), a fraction consisting of components contained in the extract, i.e., cis-isohumulone, trans-isohumulone, cis-isoadhumulone, trans-isoadhumilone, cis-isocohumulone, and trans-isocohumulone (referred to as the "purified isohumulone fraction" hereinafter), was fractionated. The water soluble extract was neutralized with hydrochloric acid and lyophilized, after which the resulting lyophilized material (3.5 g) was fractionated using silica gel chromatography (3.5 × 33 cm). The column was equilibrated and eluted with hexane:ethyl acetate (2:1). Each fraction (20 ml) of the eluate was collected using a fraction collector and their purity was confirmed using HPLC (analytical conditions were described in Reference Example). Fractions from 24 to 60 were pooled together and concentrated and dried to solid using a rotary evaporator in dark to obtain 1 g of purified isohumulone fraction consisting of cis-isohumulone, trans-isohumulone, cis-isoadhumulone, trans-isoadhumulone, cis-isocohumulone, and trans-isocohumulone. The composition ratio of each fraction based on the area ratio of the HPLC chromatogram was isocohumulone (cis-type + trans-type): isohumulone (cis-type + trans-type): isoadhumulone (cis-type + trans-type) = 50.2:27.1:22.7. C57BL/6NCrj female mice (5-weeks of age, 8 per group; Japan Charles River) were fed CE2 (Japan Clea) and water for 1 week ad libitum. Then, the animals were divided into 3 groups, i.e., a group fed AIN76A (described in Example 2) with addition of 0.2% cholesterol and 0.3% cellulose (hereinafter referred to as "group C"), a group fed AIN76A with addition of 0.2% cholesterol and 1% water soluble extract (hereinafter referred to as "group W") , and a group fed AIN76A with addition of 0.2% cholesterol and 0.3% purified isohumulone fraction described above (hereinafter referred to as "group IH"; the content of isohumulones in this diet was almost the same as that in the diet fed in group W). The diets were prepared and administered according to the methods described in Example 2. Further, in this experiment, individual animals were reared separately, and fed 3.5 g of diet per day. Further, the amount of uneaten diet was measured using a sieve and subtracted to calculate the amount of diet intake. One week after, dissection was carried out under non-fasting conditions, whole blood was collected from the abdominal vein and triglyceride was measured according to the method described in Example 1 (Figure 48). The amount of blood triglyceride significantly decreased in both group IH and group W. Further, the cholesterol, triglyceride and phospholipid contents per g of liver were measured (Figures 49, 50 and 51), which confirmed a significant decrease in the cholesterol content and a decreasing tendency in the triglyceride content in both group IH and group W. Change in body weight was shown in Figure 52 and the amount of body weight gain per calorie intake is shown in Figure 53. A significant body weight decrease in group W and significantly reduced body weight gain per calorie intake in group IH were shown. From the abovementioned Example, it was revealed that the purified isohumulone fraction was effective in improving lipid metabolism, reducing triglyceride in plasma, preventing the accumulation of cholesterol in the liver, and suppressing body weight gain.

### Example 14

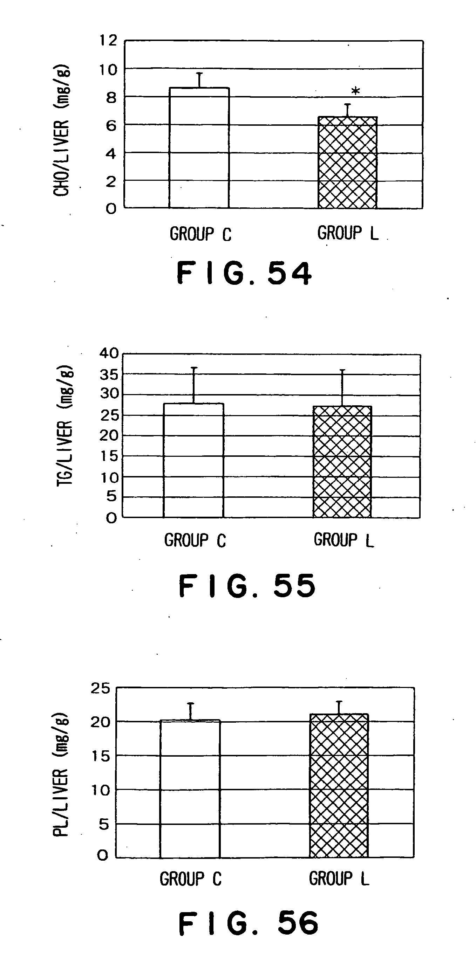

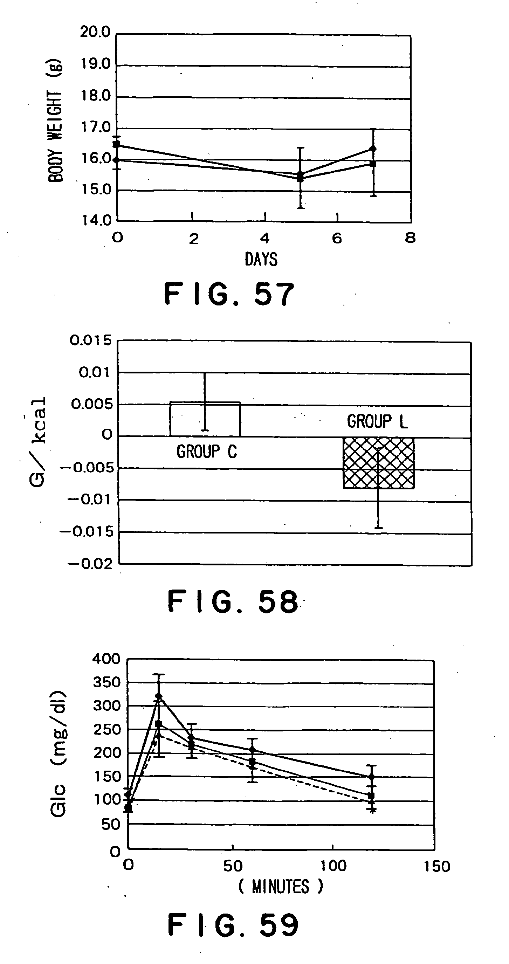

Improving effect of a fractionated lupulone on lipid metabolism was evaluated using C57BL/6 mice (females). Namely, lupulone was purified from hop pellets (CAS pellets, a product of Saaz, Czech Republic). About 2.5 kg of hop pellets were extracted with 4 L of ethyl acetate 3 times and the extract was concentrated under the reduced pressure to obtain a dark green extract (329.17 g). A portion of the extract (262.7 g) was applied on a silica gel column for fractionation. Chromatography was carried out using a stepwise elution with a hexane-ethyl acetate mixed solution to obtain 15 fractions. The third fraction (41.8 g) was applied on a silica gel column for refractionation and a fraction eluted with a hexane:ethyl acetate (20:1) solution was recrystalized to obtain lupulone (1.88 g, white needle crystals, yield: about 0.094%). Further, 5-week-old C57BL/6NCrj female mice (8 per group) (Japan Charles River) were fed CE2 (Japan Clea) and water for 1 week ad libitum. Then, the animals were divided into 2 groups, i.e., a group fed AIN76A (described in Example 2) with addition of 0.2% cholesterol and 0.3% cellulose (referred to as "group C" hereinafter) and a group fed AIN76A with addition of 0.2% cholesterol and 0.3% lupulone (referred to as "group L" hereinafter). The diets were prepared and administered according to the methods described in Example 2. One week after feeding the test diets, the animals were dissected under non-fasting conditions. The cholesterol, triglyceride and phospholipid contents per g of liver were measured (Figures 54, 55 and 56), which confirmed a significant decrease in the cholesterol content in group L. Change in body weight is shown in figure 57 and the amount of body weight gain per calorie intake is shown in Figure 58. A significant decrease in body weight and significantly reduced body weight gain per calorie intake were shown in group L. From the aforementioned Example, it was revealed that lupulone was effective in improving lipid metabolism, preventing the accumulation of cholesterol in the liver, and suppressing body weight gain.

### Example 15

C57BL/6 mice were fed the high fat diet shown in Example 8 for 12 weeks to induce insulin resistance and then orally administered with the water soluble hop extract for 10 consecutive days(100 and 330 mg/kg/day). After completion of the administration, animals were fasted for 16 hours and then subjected to an oral glucose tolerance test (OGTT). Similarly, mice in which insulin resistance was similarly induced were orally administered with a purified isocohumulone product (a mixture of cis and trans forms) prepared according to the method described in Reference Example for 10 consecutive days (10 and 30 mg/kg/day). After completion of the administration, animals were fasted for 16 hours and then subjected to an oral glucose tolerance test (OGTT). In OGTT, after blood sampling and blood sugar measurement, 1 g/kg of aqueous glucose solution was administered (at time zero), after which blood sampling and blood sugar measurement were carried out at 15, 30, and 60 minutes and blood sugar measurement was carried out art 120 minutes. Change with time in the blood insulin level was measured using an insulin measuring kit (Morinaga Seikagaku Institute) .

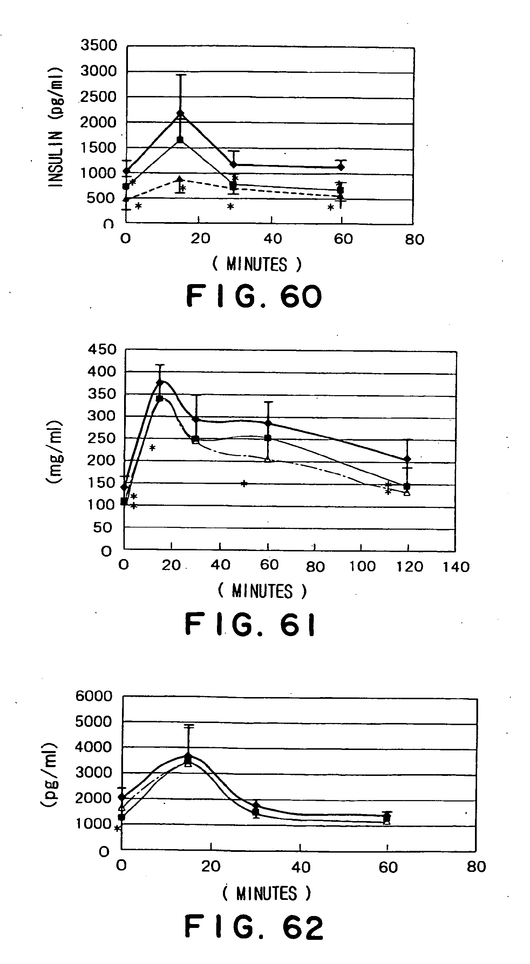

Changes in the sugar level and insulin concentration in the blood in the group administered with the water soluble extract are shown in Figures 59 and 60. Ameliorations in glucose tolerance and insulin resistance were observed in the group administered with the water soluble extract ("group W" in Figures). Changes in the sugar level and insulin concentration in the blood in the group administered with the purified isocohumulone ("Group IH" in Figures) are shown in Figures 61 and 62. Amelioration in glucose tolerance was observed in the group administered with the purified isocohumulone as in the group administered with the water soluble extract. Further, the insulin concentration before the administration significantly decreased and tended to keep decreasing thereafter, which suggested the amelioration of insulin resistance. The results above confirmed that administration of the hop extract for such a short time as 10 days ameliorated insulin resistance of mice fed high fat diet and such effect was similarly observed with the purified isocohumulone product.

### Example 16

Eighteen 8-week-old male ApoE knockout mice (imported from Jackson Laboratory) were purchased, divided into groups of nine, i.e., a group for the water soluble extract (described in Example 2) (W) and a control group (C), and fed the high fat and high cholesterol diet shown in Table 1 of Example 1 for 10 weeks. After 10 weeks, the animals were sacrificed under ether anesthesia by bleeding the abdominal vena cava. After obtaining organs such as the liver and fat, the liver was immediately frozen with liquid nitrogen. The aortae were removed along with the heart. For the aortae, the thoracic aorta and the abdominal aorta were spread out, fixed in a 10% formalin solution and then stained with Oil Red O. The aortic arch and the aortic valve were immersed and fixed in a 10% formalin solution, embedded in paraffin for round slicing, sectioned and then stained with hematoxylin-eosin and elastica van Gieson. Analyses were performed using a tabulator measuring unit VM-30 for micromeasurement (Olympus Optical Co.) for the atherosclerotic lesion area and the total blood vessel area of the Oil-Red-O-stained thoracic aorta and abdominal aorta, and the cross-sectional intima area and the cross-sectional total area of the EVG-stained aortic arch and aortic valve. The result calculations were made for the atherosclerotic lesion area ratio (= area densely stained with Oil Red O/total blood vessel area x 100) for the thoracic aorta and the abdominal aorta and the degree of intima hypertrophy (= intima area/media area, more specifically, = intima cross-sectional area/(intima-media cross-sectional area - intima cross-sectional area)). The amount of homocysteine in the plasma was measured using a homocysteine measuring agent (Yunichika) according to the attached manual. The hepatic triglyceride was measured according to the method described in Example 1.

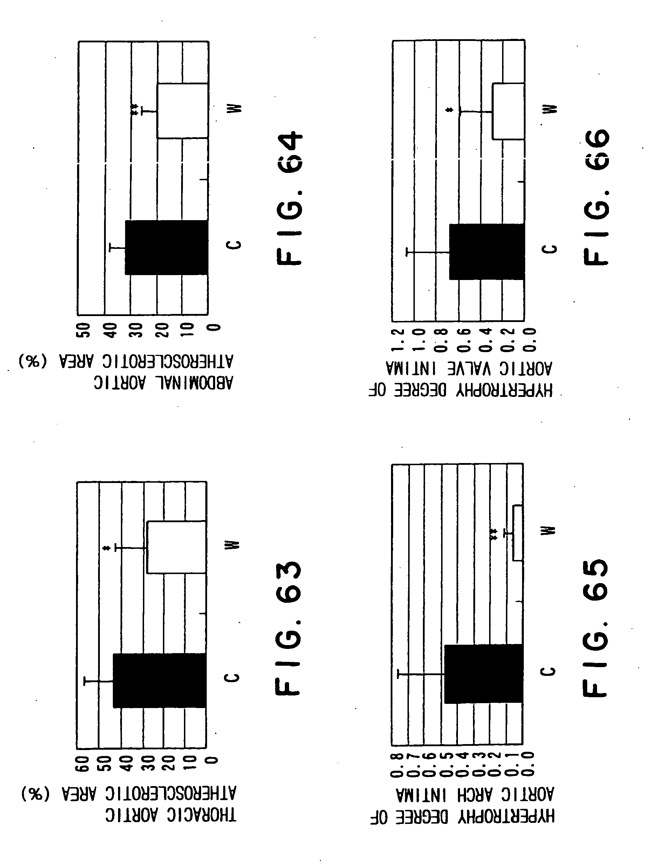

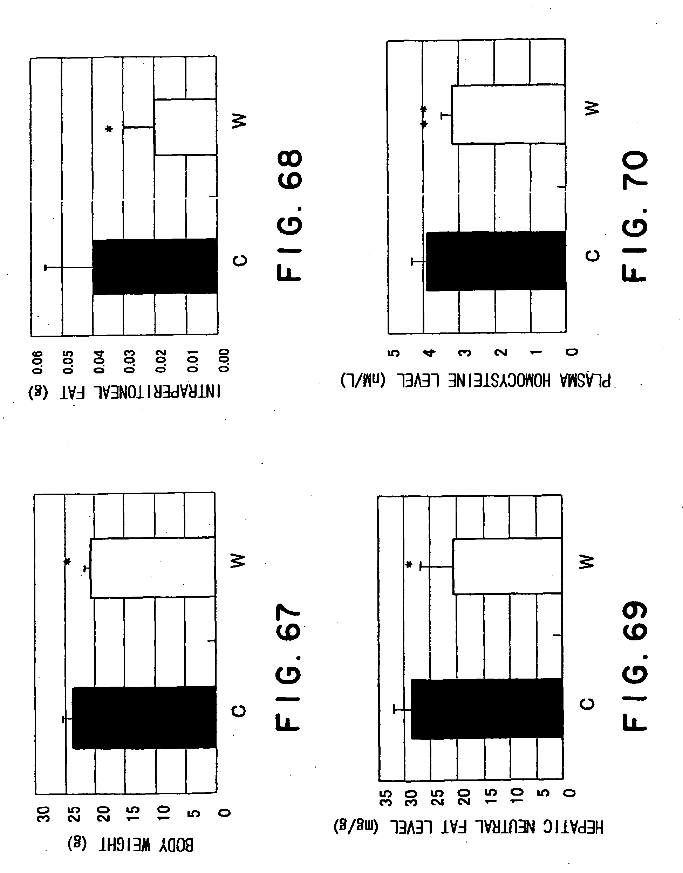

The results showed that the water soluble extract (W) reduced all the atherosclerotic lesion area of the thoracic aorta (Figure 63), the atherosclerotic lesion area of the abdominal aorta (Figure 64), the degree of intima hypertrophy in the aortic arch (Figure 65), and the degree of intima hypertrophy in the aortic valve (Figure 66). Also in group W, the body weight (Figure 67) and the intraperitoneal fat weight (Figure 68) at the time of dissection were significantly low, and a decrease in the hepatic triglyceride content was observed (Figure 69). Further, it was shown that the amount of homocysteine in the plasma was reduced by the water soluble extract (W) (Figure 70).

The results above revealed that the water soluble extract (W) mainly consisting of isohumulones has marked effects in preventing atherosclerotic changes, improving lipid metabolism, suppressing body weight gain, and suppressing the accumulation of visceral fat.

### Example 17

The effects of the hop extract and the water soluble extract on the mucous membrane of the large intestine were evaluated. The amount of PGE2 production in the mucous membrane of the large intestine in Fischer 344 rats (males) was used as an index. More specifically, 4-week-old Fischer 344 male rats (Japan Charles River) were fed AIN-76A ad libitum (described in Example 3) and water for 3 days for habituation. Then, the animals at 5 weeks of age were divided into 3 groups (4 per group) to start feeding test diets. Namely, the first group (C) was fed AIN-76A, the second group (H) was fed AIN-76A with addition of 1% hop extract (described in Example 2), and the third group (W) fed AIN-76A with addition of 1% water soluble extract (described in Example 2). One week after, the large intestine was extracted by dissection and cut longitudinally after washing out intestinal contents with physiological saline. The mucosal tissue of the large intestine was shaved off with a slide glass (Matsunami) and suspended in 500 µl of PBS. This mucosal tissue was mashed using a homogenizer and centrifuged at 10000 g for 5 minutes and the supernatant was subjected to PGE2 measurement. The PGE2 was quantitatively measured using a prostaglandin E2 enzyme immunoassay system (Amersham Pharmacia Biotech, produce code: RPN222) according to the instruction.

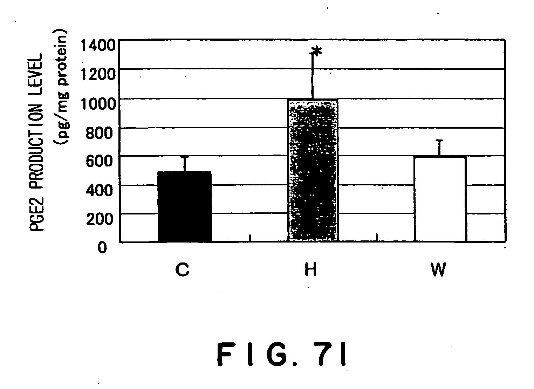

As a result, a significant increase in PGE2 production was observed in the group fed with addition of 1% hop extract (H) but not in the group fed with addition of 1% water soluble extract (W) (Figure 71). Further, enlargement of the cecum and diarrhea were observed in the group fed with addition of 1% hop extract (H).

The results above revealed that when used at high concentrations, inflammations observed in animals fed with addition of the hop extract consisting mainly humulones were not observed in animals fed with addition of the water soluble extract mainly consisting of isohumulones.

## Claims

1. A pharmaceutical composition for use in the treatment, prophylaxis, or amelioration of diabetes, diabetic complications, lipid metabolism abnormalities, hyperlipidemia, insulin resistance or diseases associated therewith, obesity, or body weight gain, comprising
a compound of formula (II) 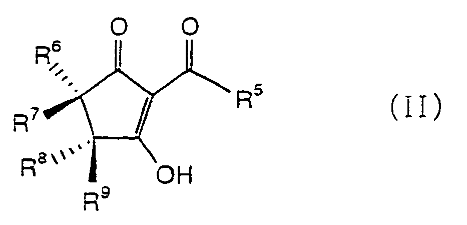 wherein R⁵, R⁶ and R⁷ represent a hydrogen atom, C₁₋₆ alkyl or C₂₋₆ alkenyl, R⁸ and R⁹ represent a hydrogen atom, a hydroxyl group, C₁₋₆ alkyl, C₂₋₆ alkenyl, -C(=O)R¹⁰, or-CH(-OH)R¹⁰, and R¹⁰ represents C₁₋₅ alkyl or C₂₋₅ alkenyl, provided that R⁸ and R⁹ do not simultaneously represent a hydroxyl group;
a compound of formula (III) 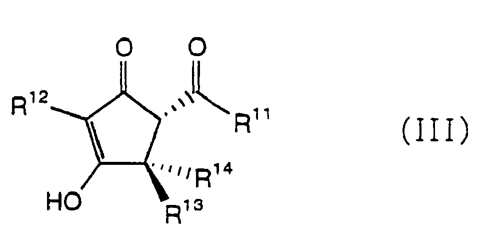 wherein R¹¹ and R¹² represent a hydrogen atom, C₁₋₆ alkyl or C₂₋₆ alkenyl, R¹³ and R¹⁴ represent a hydroxyl group, C₁₋₆ alkyl , C₂₋₆ alkenyl, -C (=O) R¹⁵, or -CH (-OH) R¹⁵, and R¹⁵ represents C₁₋₆ alkyl or C₂₋₆ alkenyl , provided that R¹³ and R¹⁴ do not simultaneously represent a hydroxyl group;
a compound of formula (IV) 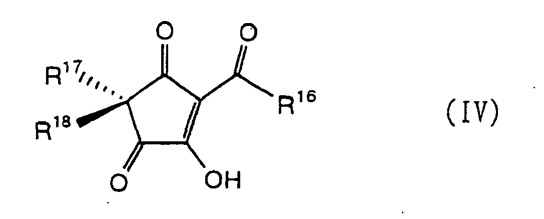 wherein R¹⁶, R¹⁷ and R¹⁸ represent a hydrogen atom, C₁₋₆ alkyl or C₁₋₆ alkenyl; or
a compound of formula (V) 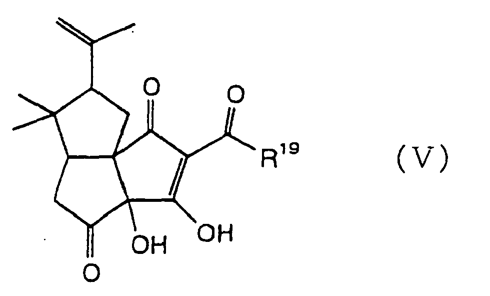 wherein R¹⁹ represents C₁₋₆ alkyl or C₂₋₆ alkenyl;
or a pharmaceutically acceptable salt or solvate thereof ; or an isomerized hop extract, as an active ingredient.

2. A composition for use in the amelioration of insulin resistance, the improvement of lipid metabolism, the suppression of body weight gain, or the slimming, comprising a compound or a pharmaceutically acceptable salt or solvate thereof or an isomerized hop extract as claimed in claim 1, as an active ingredient.

3. The composition according to any one of claims 1 to or 2, wherein said composition is provided in a form of food.

4. A food for use in the amelioration of insulin resistance, the improvement of lipid metabolism, the suppression of body weight gain, or the slimming, comprising a compound or a pharmaceutically acceptable salt or solvate thereof or an isomerized hop extract as claimed in claim 1, as an active ingredient.

5. The food as claimed in claim 4, wherein said food is a health food, a functional food, a food for specified health use, or a food for patients.

6. The food as claimed in claim 4 or 5, wherein said composition is provided in a form of food.

7. Use of a compound or a pharmaceutically acceptable salt or solvate thereof or an isomerized hop extract as claimed in claim 1 for the manufacture of a medicine for use in the treatment, prophylaxis, or amelioration of diabetes, diabetic complications, lipid metabolism abnormalities, hyperlipidemia insulin resistance or diseases associated therewith , obesity, or body weight gain.

8. Use of a compound or a pharmaceutically acceptable salt or solvate thereof or an isomerized hop extract as claimed in claim 1 for the manufacture of a composition for use in the amelioration of insulin resistance, the improvement of lipid metabolism, the suppression of body weight gain, or the slimming.

9. Use as claimed in claim 7 or 8 wherein the active ingredient is administered in a form of food.

## Patentansprüche

1. Pharmazeutische Zusammensetzung zur Verwendung in der Behandlung, Prophylaxe oder Linderung von Diabetes, diabetischen Komplikationen, Lipidmetabolismusanomalien, Hyperlipidämie, Insulinresistenz oder Erkrankungen die damit verbunden sind, Fettleibigkeit oder Köpergewichtszunahme, die folgendes umfasst:
eine Verbindung der Formel (II) 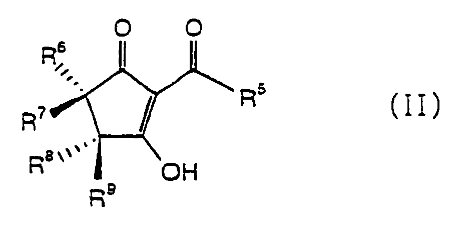 worin R⁵, R⁶ und R⁷ ein Wasserstoffatom, C₁₋₆-Alkyl oder C₂₋₆-Alkenyl ist, R⁸ und R⁹ ein Wasserstoffatom, eine Hydroxylgruppe, C₁₋₆-Alkyl, C₂₋₆-_{A}lkenyl, -C(=O)R¹⁰ oder -CH(-OH)R¹⁰ ist und R¹⁰ C₁-₆-Alkyl oder C₂₋₆-Alkenyl ist, vorausgesetzt, dass R⁸ und R⁹ nicht gleichzeitig eine Hydroxylgruppe ist;
eine Verbindung der Formel (III) 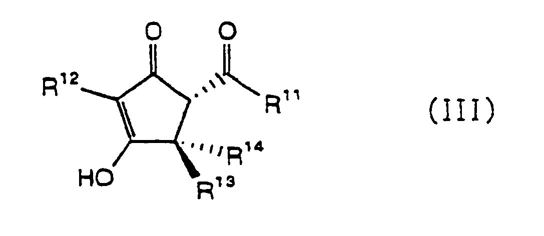 worin R¹¹ und R¹² ein Wasserstoffatom, C₁₋₆-Alkyl oder C₂₋₆-Alkenyl ist, R¹³ und R¹⁴ eine Hydroxylgruppe, C₁₋₆-Alkyl, C₂₋₆-Alkenyl, -C(=O)R¹⁵ oder -CH(-OH)R¹⁵ ist und R¹⁵ C₁₋₆-Alkyl oder C₂₋₆-Alkenyl ist, vorausgesetzt, dass R¹³ und R¹⁴ nicht gleichzeitig eine Hydroxylgruppe ist;
eine Verbindung der Formel (IV) 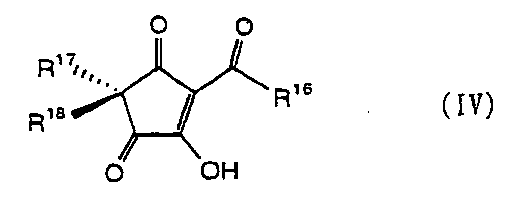 worin R¹⁶, R¹⁷ und R¹⁸ ein Wasserstoffatom, C₁₋₆-Alkyl oder C₂₋₆-Alkenyl ist; oder
eine Verbindung der Formel (V) 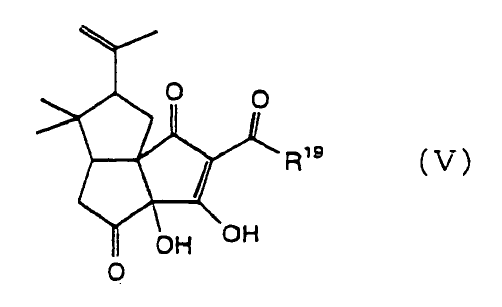 worin R¹⁹ C₁₋₆₋Alkyl oder C₂₋₆-Alkenyl ist;
oder ein pharmazeutisch akzeptables Salz oder Solvat davon; oder ein isomerisiertes Hopfenextrakt als wirksamen Bestandteil

2. Zusammensetzung zur Verwendung in der Linderung von Insulinresistenz, zur Verbesserung des Lipidmetabolismus, zur Unterdrückung der Körpergewichtszunahme oder der Abnahme, die eine Verbindung oder ein pharmazeutisch akzeptables Salz oder Solvat davon oder ein isomerisiertes Hopfenextrakt gemäß Anspruch 1 als wirksamen Bestandteil umfasst.

3. Zusammensetzung gemäß irgendeinem der Ansprüche 1 oder 2, worin die Zusammensetzung in Form eines Nahrungsmittels bereitgestellt ist.

4. Nahrungsmittel zur Verwendung in der Linderung von Insulinresistenz, der Verbesserung des Lipidmetabolismus, der Unterdrückung der Körpergewichtszunahme oder der Abnahme, das eine Verbindung oder ein pharmazeutisch akzeptables Salz oder Solvat davon oder ein isomerisiertes Hopfenextrakt gemäß Anspruch 1 als wirksamen Bestandteil umfasst.

5. Nahrungsmittel gemäß Anspruch 4, worin das Nahrungsmittel ein Gesundheitsnahrungsmittel, ein funktionelles Nahrungsmittel, ein Nahrungsmittel zur speziellen Gesundheitsverwendung oder ein Nahrungsmittel für Patienten ist.

6. Nahrungsmittel gemäß Anspruch 4 oder 5, worin die Zusammensetzung in Form eines Nahrungsmittels bereitgestellt ist.

7. Verwendung einer Verbindung oder eines pharmazeutisch akzeptablen Salzes oder Solvates davon oder eines isomerisierten Hopfenextraktes gemäß Anspruch 1 zur Herstellung eines Medikaments für die Verwendung in der Behandlung, Prophylaxe oder Linderung von Diabetes, diabetischen Komplikationen, Lipidmetabolismusanomalien, Hyperlipidämie, Insulinresistenz oder Erkrankungen die damit verbunden sind, Fettleibigkeit oder Körpergewichtszunahme.

8. Verwendung einer Verbindung oder eines pharmazeutisch akzeptablen Salzes oder Solvates davon oder eines isomerisierten Hopfenextraktes gemäß Anspruch 1 zur Herstellung einer Zusammensetzung für die Verwendung in der Linderung von Isulinresistenz, der Verbesserung des Lipidmetabolismus, der Unterdrückung der Gewichtszunahme oder der Abnahme.

9. Verwendung gemäß Anspruch 7 oder 8, worin der wirksame Bestandteil in Form eines Nahrungsmittels verabreicht wird.

## Revendications

1. Composition pharmaceutique destinée au traitement, à la prophylaxie, ou à l'amélioration du diabète, des complications du diabète, des anomalies du métabolisme lipidique, de l'hyperlipidémie, de la résistance à l'insuline ou des maladies qui lui sont associées, de l'obésité, ou de la prise de poids, comprenant
un composé de formule (II) 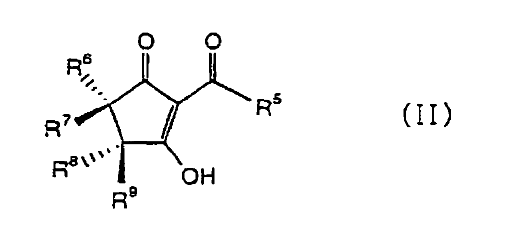 dans laquelle R⁵, R⁶ et R⁷ représentent un atome d'hydrogène, un alkyle C₁₋₆ ou un alcényle C₂₋₆, R⁸ et R⁹ représentent un atome d'hydrogène, un groupe hydroxyle, un alkyle C₁₋₆ ou un alcényle C₂₋₆, - C(=O)R¹⁰, ou -CH(-OH)R¹⁰, et R¹⁰ représente un alkyle C₁₋₆ ou un alcényle C₂₋₆, à condition que R⁸ et R⁹ ne représentent pas simultanément un groupe hydroxyle ;
un composé de formule (III) 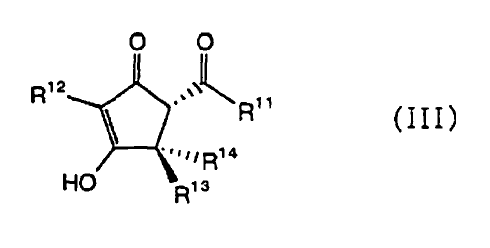 dans laquelle R¹¹ et R¹² représentent un atome d'hydrogène, un alkyle C₁₋₆ ou un alcényle C₂₋₆, R¹³ et R¹⁴ représentent un groupe hydroxyle, un alkyle C₁₋₆, un alcényle C₂₋₆, -C(=O)R¹⁵, ou -CH(-OH)R¹⁵, et R¹⁵ représente un alkyle C₁₋₆ ou un alcényle C₂₋₆, à condition que R¹³ et R¹⁴ ne représentent pas simultanément un groupe hydroxyle ;
un composé de formule (IV) 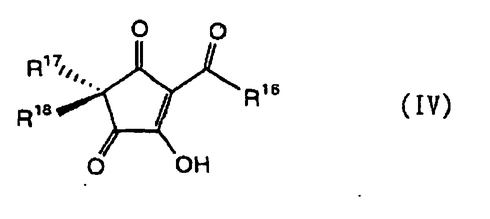 dans laquelle R¹⁶, R¹⁷ et R¹⁸ représentent un atome d'hydrogène, un alkyle C₁₋₆ ou un alcényle C₂₋₆ ; ou
un composé de formule (V) 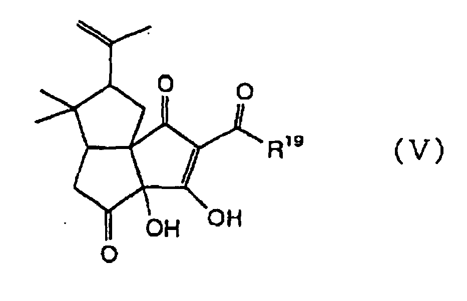 dans laquelle R¹⁹ représente un alkyle C₁₋₆ ou un alcényle C₂₋₆ ;
ou un sel ou solvate de celui-ci pharmaceutiquement acceptable ; ou un extrait de houblon isomérisé, à titre de principe actif.

2. Composition destinée à l'amélioration de la résistance à l'insuline, l'amélioration du métabolisme lipidique, la suppression de la prise de poids, ou l'amincissement, comprenant un composé ou un sel ou solvate de celui-ci pharmaceutiquement acceptable ou un extrait de houblon isomérisé selon la revendication 1, à titre de principe actif.

3. Composition selon l'une quelconque des revendications 1 ou 2, dans laquelle ladite composition est fournie sous la forme d'un aliment.

4. Aliment destiné à l'amélioration de la résistance à l'insuline, l'amélioration du métabolisme lipidique, la suppression de la prise de poids, ou l'amincissement, comprenant un composé ou un sel ou solvate de celui-ci pharmaceutiquement acceptable ou un extrait de houblon isomérisé selon la revendication 1, à titre de principe actif.

5. Aliment selon la revendication 4, dans lequel ledit aliment est un aliment diététique, un aliment fonctionnel, un aliment à visée diététique spécifiée, ou un aliment destiné à des patients.

6. Aliment selon les revendications 4 ou 5, dans lequel ladite composition est fournie sous la forme d'un aliment.

7. Utilisation d'un composé ou d'un sel ou solvate de celui-ci pharmaceutiquement acceptable ou d'un extrait de houblon isomérisé selon la revendication 1 dans la fabrication d'un médicament destiné au traitement, à la prophylaxie, ou à l'amélioration du diabète, des complications du diabète, des anomalies du métabolisme lipidique, de l'hyperlipidémie, de la résistance à l'insuline ou des maladies qui lui sont associées, de l'obésité, ou de la prise de poids.

8. Utilisation d'un composé ou d'un sel ou solvate de celui-ci pharmaceutiquement acceptable ou d'un extrait de houblon isomérisé selon la revendication 1 dans la fabrication d'une composition destinée à l'amélioration de la résistance à l'insuline, l'amélioration du métabolisme lipidique, la suppression de la prise de poids, ou l'amincissement.

9. Utilisation selon les revendications 7 ou 8, dans laquelle le principe actif est administré sous la forme d'un aliment.
